# EUROPÄISCHE PATENTSCHRIFT

(11) **EP 1 420 771 B1**
(45) Veröffentlichungstag und Bekanntmachung des Hinweises auf die Patenterteilung: **06.09.2006**
(21) Anmeldenummer: 02764680.1
(22) Anmeldetag: 12.07.2002
(51) Int. Cl.: A61K 31/00, A61K 31/12, A61K 31/122, C07C 49/255, C07C 49/233, C07C 49/35, C07C 49/333, A61P 3/04, A61P 3/00, A61P 9/12, A61P 3/10, A61P 3/06

(54) **VERWENDUNG TRIFLUORACETYLALKYL-SUBSTITUIERTER PHENYL-, PHENOL- UND BENZOYLDERIVATE IN DER BEHANDLUNG UND/ODER PROPHYLAXE VON OBESITAS UND DEREN BEGLEIT- UND/ODER FOLGEERKRANKUNGEN**
USE OF TRIFLUOROACETYL ALKYL-SUBSTITUTED PHENYL DERIVATIVES, PHENOL DERIVATIVES, AND BENZOYL DERIVATIVES IN THE TREATMENT AND/OR PROPHYLAXIS OF OBESITY AND ACCOMPANYING DISEASES AND/OR SECONDARY DISEASES
UTILISATION DE DERIVES DE PHENYLE, DE PHENOL ET DE BENZOYLE SUBSTITUES PAR TRIFLUOROACETYLALKYLE DANS LE TRAITEMENT ET LA PROPHYLAXIE DE L'OBESITE DE SES MALADIES ASSOCIEES ET/OU SECONDAIRES ACCOMPAGNANT L'OBESITE

(30) Priorität: 18.07.2001 DE 10135027
(43) Veröffentlichungstag der Anmeldung: 26.05.2004
(73) Patentinhaber: Solvay Pharmaceuticals GmbH, 30173 Hannover (DE)
(72) Erfinder: ANTEL, Jochen, 31848 Bad Münder (DE); WALDECK, Harald, 30916 Isernhagen (DE); POTTHOFF, Andreas, 30171 Hannover (DE); EYTING, Sabine, 48159 Münster (DE); GREGORY, Peter-Colin, 30559 Hannover (DE)
(74) Vertreter: Bauriegel, Lutz
(86) Internationale Anmeldenummer: PCT/EP2002/007782
(87) Internationale Veröffentlichungsnummer: WO 2003/007923

(56) Entgegenhaltungen:
- EP-A- 0 289 390
- EP-A- 0 354 548
- EP-A- 0 611 232
- EP-A- 1 167 355
- WO-A-00/40569
- WO-A-02/46129
- WO-A-89/04819
- WO-A-98/23605
- WO-A-99/15129
- FR-A- 2 098 305
- US-A- 3 046 300
- US-A- 4 297 515
- US-A- 4 408 077
- DATABASE BIOSIS [Online] BIOSCIENCES INFORMATION SERVICE, PHILADELPHIA, PA, US; 1990 BURTSEV E M ET AL: "FACTORS FOR THE RISK OF ACUTE AND CHRONIC COURSE OF CEREBRAL ISCHEMIAS" Database accession no. PREV199090072386 XP002244872 & ZHURNAL NEVROPATOLOGII I PSIKHIATRII IMENI S S KORSAKOVA, Bd. 90, Nr. 1, 1990, Seiten 16-20, ISSN: 0044-4588
- HAN CHANG ET AL: "85-KDA cytosolic phospholipase A2 plays a critical role in PPAR-mediated gene transcription in human hepatoma cells." HEPATOLOGY, Bd. 34, Nr. 4 Pt. 2, Oktober 2001 (2001-10), Seite 495A XP008018675 52nd Annual Meeting and Postgraduate Courses of the American Association for the Study of Liver Diseases;Dallas, Texas, USA; November 09-13, 2001, hDB&searchDBfor=home&id=jhep October, 2001 ISSN: 0270-9139
- BOGER, DALE L. ET AL: "Trifluoromethyl ketone inhibitors of Fatty Acid Amide Hydrolase: a probe of structural and conformational features contributing to inhibition" BIOORGANIC & MEDICINAL CHEMISTRY LETTERS (1999), 9(2), 265-270 , XP004152614
- KAWASE, MASAMI ET AL: "Trifluoromethyl ketone-based inhibitors of apoptosis in cerebellar granul neurons" BIOLOGICAL & PHARMACEUTICAL BULLETIN (2001), 24(11), 1335-1337 , XP008013056
- ALLEN, KAREN N. ET AL: "Inhibition of pig liver esterase by trifluoromethyl ketones: modulators of the catalytic reaction alter inhibition kinetics" BIOCHEMISTRY (1989), 28(1), 135-40 , XP001134948
- GHOMASHCHI F ET AL: "Trifluoromethyl ketones and methyl fluorophosphonates as inhibitors of group IV and VI phospholipases A2: structure-function studies with vesicle, micelle, and membrane assays" BIOCHIMICA ET BIOPHYSICA ACTA. BIOMEMBRANES, AMSTERDAM, NL, Bd. 1420, Nr. 1-2, 20. August 1999 (1999-08-20), Seiten 45-56, XP004273020 ISSN: 0005-2736
- YANG, DAN ET AL: "Regioselective Intramolecular Oxidation of Phenols and Anisoles by Dioxiranes Generated in Situ" JOURNAL OF ORGANIC CHEMISTRY (2000), 65(13), 4179-4184 , XP002244869
- BEGUE, JEAN PIERRE ET AL: "The Wittig reaction of perfluoro acid derivatives: access to fluorinated enol ethers, enamines, and ketones" JOURNAL OF ORGANIC CHEMISTRY (1992), 57(14), 3807-14 , XP002244870
- HAMMOCK, BRUCE D. ET AL: "Trifluoromethylketones as possible transition state analog inhibitors of juvenile hormone esterase" PESTICIDE BIOCHEMISTRY AND PHYSIOLOGY (1982), 17(1), 76-88 , XP008013015
- PINDER, ROGER M. ET AL: "2-Amino-3-phenyl-1,1,1-trifluoropropanes. Fluorine analogs of amphetamines" JOURNAL OF MEDICINAL CHEMISTRY (1969), 12(2), 322-4 , XP002244871
- BIED, C. ET AL: "Synthesis and reactivity of benzylic and allylic samarium compounds" TETRAHEDRON (1992), 48(19), 3877-90 , XP001109525
- CHENG, C. H. ET AL: "Polymers containing fluorinated ketone groups. II. NMR studies of the reaction of methylbenzyl trifluoromethyl ketones with alcohols in carbon tetrachloride" JOURNAL OF POLYMER SCIENCE, POLYMER CHEMISTRY EDITION (1980), 18(6), 1877-82 , XP001109522
- BOIVIN J ET AL: "AN EXPEDIENT ACCESS TO TRIFLUOROMETHYL KETONES FROM CARBOXYLIC ACIDS" TETRAHEDRON LETTERS, ELSEVIER SCIENCE PUBLISHERS, AMSTERDAM, NL, Bd. 33, Nr. 10, 3. März 1992 (1992-03-03), Seiten 1285-1288, XP000262117 ISSN: 0040-4039 in der Anmeldung erwähnt
- WIEDEMANN J. ET AL.: "Direct preparation of trifluoromethyl ketones from carboxylic esters: trifluoromethylation with (trifluoromethyl)trimethylsilane" ANGEW. CHEM. INT. ED. ENGL., Bd. 37, Nr. 6, 1998, Seiten 820-821, XP001109527
- KIM S ET AL: "Radical-mediated synthesis of trifluoroethyl amines and trifluoromethyl ketones from alkyl iodides" TETRAHEDRON LETTERS, ELSEVIER SCIENCE PUBLISHERS, AMSTERDAM, NL, Bd. 43, Nr. 40, 30. September 2002 (2002-09-30), Seiten 7189-7191, XP004385616 ISSN: 0040-4039
- KAWASE M ET AL: "alpha-Trifluoromethylated Acyloins Induce Apoptosis in Human Oral Tumor Cell Lines" BIOORGANIC & MEDICINAL CHEMISTRY LETTERS, OXFORD, GB, Bd. 9, Nr. 21, 1. November 1999 (1999-11-01), Seiten 3113-3118, XP004181017 ISSN: 0960-894X
- REID J C ET AL: "SOME NEW BETA-DIKETONES CONTAINING THE TRIFLUOROMETHYL GROUP" JOURNAL OF THE AMERICAN CHEMICAL SOCIETY, AMERICAN CHEMICAL SOCIETY, WASHINGTON, DC, US, Bd. 72, Nr. 7, 1. Juli 1950 (1950-07-01), Seiten 2948-2952, XP000654013 ISSN: 0002-7863

## Beschreibung

Die vorliegende Erfindung betrifft die Verwendung neuer und bekannter Trifluoracetyl-substituierter Phenyl-, Phenol- und Benzoylderivate zur Herstellung pharmazeutischer Zubereitungen für die Behandlung und/oder Prophylaxe von Obesitas sowie von der damit einhergehenden Begleit- und/oder Folgeerkrankung, dem Metabolischen Syndrom.

Ferner betrifft die Erfindung neue Trifluoracetyl-substituierte Phenyl-, Phenol- und Benzoylderivate, diese enthaltende pharmazeutische Zubereitungen sowie Verfahren zur Herstellung dieser Verbindungen. Die erfindungsgemäß verwendeten Verbindungen wirken dabei als Inhibitoren der Lipase, insbesondere der Pankreaslipase.

Aus der EP 0 129 748 A1 sind bereits Hexadecansäure- und Hexadecadiensäurederivate bekannt, welche die Pankreaslipase hemmen und daher bei der Bekämpfung oder Verhütung von Obesitas und Hyperlipämien verwendet werden können.

Aus der WO 99/34786 sind bereits Lipase-inhibierende Polymere bekannt, welche zur Behandlung von Obesitas geeignet sind.

In der WO 00/40247 und in der WO 00/40569 werden 2-substituierte 4H-3,1-Benzoxazin-4-on-derivate beschrieben, welche als Lipase-Inhibitoren wirken und welche zur Behandlung von Obesitas eingesetzt werden können.

Ferner werden in der WO 99/15129 selektive cPLA₂-Inhibitoren beschrieben, welche unter..anderem auch gewisse Trifluoracetylalkyl-substituierte Arylderivate mitumfassen. In Biochim. Biophys. Acta, 1420 (1999), 45-56 (Gomashchi) wird die Verwendungs von weiteren Trifluoracetylalkyl-substituierten Phenylderivaten als iPLA₂-Hemmern offenbart.

Aufgabe der vorliegenden Erfindung war es, neue Lipase-inhibitorisch wirksame Arzneimittel zur Behandlung und/oder Prophylaxe von Obesitas sowie deren Begleit- und/oder Folgeerkrankung, dem Metabolischen Syndrom zur Verfügung zu stellen, welche gut wirksam sind und auf einfachem Wege erhalten werden können.

Es wurde nun gefunden, daß eine Gruppe von Trifluoracetylalkyl-substituierten Phenyl-, Phenol- und Benzoylderivaten als Inhibitoren der Lipase, insbesondere der Pankreaslipase, wirken können. Die erfindungsgemäß verwendeten Verbindungen sind somit in der Lage, die durch Pankreaslipase induzierte Fettverdauung bei Säugetieren, insbesondere Menschen, herabzusetzen, mit der Folge, daß dem Körper insgesamt weniger verwertbare Nahrungsfette zur Verfügung stehen. Die erfindungsgemäß verwendeten Verbindungen erscheinen daher geeignet zur Behandlung und/oder Prophylaxe von Obesitas und der damit im Zusammenhang stehenden Erkrankung, dem Metabolischen Syndrom.

Erfindungsgemäß werden als Lipase-inhibitorisch wirksame Wirkstoffe Trifluoracetylalkyl-substituierte Phenyl-, Phenol- und/oder Benzoylderivate der allgemeinen Formel I verwendet, 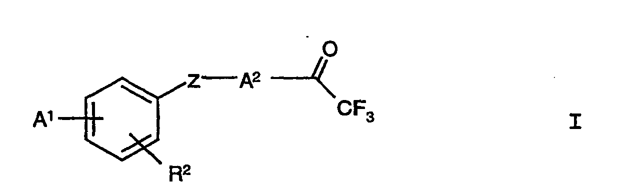 worin
- A¹: eine Gruppe der Formel R¹-W-A³-Y-(CH₂)ₙ- bedeutet, worin
R¹ Wasserstoff,
C₁₋₄-Alkyl
C₃₋₇-Cycloalkyl,
Phenyl-C₀₋₄-alkyl, welches im Phenylring gegebenenfalls durch C₁₋₄-Alkylendioxy oder ein- bis zweifach durch C₁₋₄Alkyl, C₁₋₄Alkoxy, Halogen oder Perfluor -C₁₋₄-Alkyl substituiert ist, oder
Naphthyl bedeutet,
W eine Bindung oder Sauerstoff bedeutet,
A³ eine Bindung oder C₁₋₂₀-Alkylen bedeutet, welches gegebenenfalls ein- bis zweifach durch Phenyl, Naphthyl, C₁₋₄-Alkyl oder C₅₋₇-Cycloalkyl substituiert ist,
Y eine Bindung oder Sauerstoff bedeutet und
n eine ganze Zahl von 0 bis 3 bedeutet, und

- R²: Wasserstoff, C₁₋₄-Alkyl, C₁₋₄-Alkoxy oder Halogen bedeutet, oder
- A¹ und R²: gemeinsam mit den Kohlenstoffatomen, an welche sie gebunden sind, eine C₅₋₇-Cycloalkylgruppe bilden, deren sp³-hybridisierte Kohlenstoffatome gegebenenfalls ein- bis zweifach durch Sauerstoff ersetzt sind,
- Z: eine Bindung, Sauerstoff oder Carbonyl bedeutet und
- A²: C₁₋₂₀-Alkylen bedeutet, welches gegebenenfalls einfach durch C₁₋₁₂-Alkyl, C₁₋₁₂-Alkyl-phenyl oder C₁₋₁₂-Alkyloxyphenyl substituiert ist, bedeutet,
sowie von Solvaten und/oder Hydraten der Verbindungen der Formel I und von Gemischen dieser Verbindungen, zur Herstellung pharmazeutischer Zubereitungen für die Behandlung und/oder Prophylaxe von Obesitas sowie deren Begleit- und/oder Folgeerkrankung dem Metabolischen Syndrom.

Sofern in Verbindungen der Formel I oder in anderen im Rahmen der vorliegenden Erfindung beschriebenen Verbindungen Substituenten C₁₋₄-Alkyl bedeuten oder enthalten, kann dieses geradkettig oder verzweigt sein.

Sofern Substituenten Halogen bedeuten oder enthalten, kommen insbesondere Fluor, Chlor oder Brom in Frage.

Sofern in der Gruppe A¹ der Substituent R¹ für gegebenenfalls im Phenylring substituiertes Phenyl-C₀₋₄-alkyl steht, sind unsubstituierte Phenylringe bevorzugt. Sofern R¹ Perfluor-C₁₋₄-Alkyl enthält, ist Trifluormethyl bevorzugt. Besonders bevorzugte Bedeutungen für R¹ sind Wasserstoff, C₁₋₄-Alkyl, insbesondere Methyl, C₃₋₇-Cycloalkyl, insbesondere Cyclohexyl, Phenyl und Naphthyl.

A³ steht vorzugsweise für eine Bindung oder für unverzweigtes C₁₋₂₀-, insbesondere C₁₋₇-Alkylen. n steht vorzugsweise für eine ganze Zahl von 0 bis 1.

Der Substituent R² steht vorzugsweise für Wasserstoff oder für Halogen, insbesondere Brom. Sofern R² für C₁₋₄-Alkoxy steht, ist Methoxy bevorzugt.

Z bedeutet vorzugsweise Sauerstoff oder Carbonyl, insbesondere Carbonyl.

A² bedeutet vorzugsweise n-Propylen, welches insbesondere nicht substituiert ist. Sofern eine Alkylenkette A² durch C₁₋₁₂-Alkyl, C₁₋₁₂-Alkyl-phenyl oder C₁₋₁₂-Alkyl-oxyphenyl substituiert ist, kommen insbesondere n-C₁₋₁₂-Alkylgruppen der Substituenten in Frage. Sofern Z für Carbonyl steht und A² einfach durch C₁₋₁₂-Alkyl, C₁₋₁₂-Alkyl-phenyl oder C₁₋₁₂-Alkyl-oxyphenyl substituiertes C₁₋₂₀-Alkylen bedeutet, ist vorzugsweise das an die Carbonylgruppe Z gebundene Kohlenstoffatom der Gruppe A² substituiert.

Verbindungen der Formel I, worin Substituenten, insbesondere die Gruppe A¹, in para-Stellung zu dem Rest "-Z- A²⁻C(O)-CF₃" angeordnet sind, sind bevorzugt.

Bevorzugte Verbindungen der Formel I sind 1,1,1-Trifluor-9-phenyl-nonan-2-on und 1,1,1-Trifluor-8-phenyloctan-2-on.

In einer Ausführungsform der Erfindung werden Verbindungen der allgemeinen Formel If verwendet, 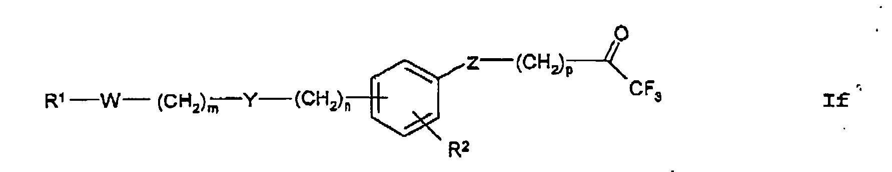 worin R¹, R², W, Y und Z obige Bedeutungen besitzen, m eine ganze Zahl von 0 bis 10 bedeutet, n eine ganze Zahl von 0 bis 3 bedeutet und p eine ganze Zahl von 1 bis 20 bedeutet.

Die Verbindungen 5-[4-(Benzyloxymethyl)-phenoxy]-1,1,1-trifluorpentan-2-on; 5-[4-(Benzyloxy)phenoxy]-1,1,1-trifluorpentan-2-on; 1,1,1-Trifluor-12-phenoxy-dodecan-2-on und 1,1,1-Trifluor-5-[4-(3-phenylpropoxy)phenoxy]pentan-2-on sind neue Verbindungen und stellen eine erste Ausführungsform der Erfindung dar.

Die Verbindung 6-(4-Methoxyphenyl)-1,1,1-trifluorhexan-2-on ist eine neue Verbindung und stellt eine zweite Ausführungsform der Erfindung dar.

Die Verbindung 1,1,1-Trifluor-11-phenyl-undecan-2-on ist eine neue Verbindung und stellt eine dritte Ausführungsform der Erfindung dar.

Eine besonders bevorzugte Ausführungsform der Erfindung stellen die Verbindungen der allgemeinen Formel Ig dar, 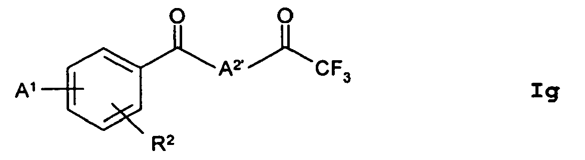 worin A¹ und R² obige Bedeutungen besitzen und A^{2'} n-Propylen bedeutet, welches gegebenenfalls einfach durch C₁₋₁₂-Alkyl, C₁₋₁₂-Alkyl-phenyl oder C₁₋₁₂-Alkyl-oxyphenyl substituiert ist, sowie die Solvate und Hydrate von Verbindungen der Formel Ig. Verbindungen der Formel Ig sind neue Verbindungen. Bevorzugte Verbindungen, welche unter die Formel Ig fallen, sind beispielsweise 6,6,6-Trifluor-1-(4-methoxyphenyl)hexan-1,5-dion; 6,6,6-Trifluor-1-(4-(4-phenoxybutoxy)phenyl)hexan-1,5-dion; 6, 6, 6-Trifluor-1- (4- (3-phenylpropoxy) phenyl) hexan-1,5-dion; 1-(4-Bromphenyl)-6,6,6-trifluorhexan-1,5-dion; 6,6,6-Trifluor-1-(4-(1-naphthyl)phenyl)hexan-1,5-dion; 6,6,6-Trifluor-1-(5,6,7,8-tetrahydronaphthalen-2-yl)hexan-1,5-dion; 6,6,6-Trifluor-1-(4-(9-methoxy-1-naphthyl)phenyl)hexan-1,5-dion; 6, 6, 6-Trifluor-1-(4-(2-naphthyl) phenyl) hexan-1,5-dion; 6,6,6-Trifluor-1-(4-(hexadecyloxy)phenyl)hexan-1,5-dion und 6,6,6-Trifluor-1-(4-(tetradecyloxy)phenyl)hexan-1,5-dion. Eine Untergruppe der Verbindungen der Formel Ig stellen die Verbindungen der allgemeinen Formel Id dar, 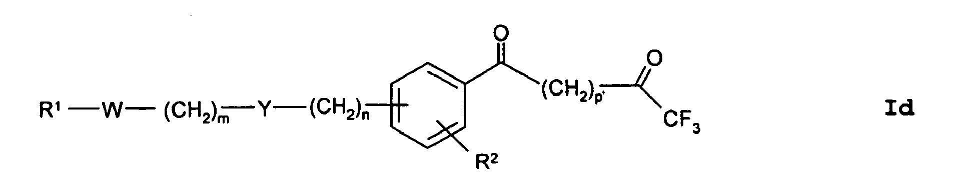 worin R¹, R², W, Y, m, und n obige Bedeutungen besitzen und
worin p' 3 bedeutet.

Verbindungen, welche ausgewählt sind aus der Gruppe bestehend aus 1,1,1-Trifluor-7-phenyl-heptan-2-on und 5-(4-Methoxyphenyl)-1,1,1-trifluorpentan-2-on, sind bereits an sich bekannt, beispielsweise aus der EP 0 434 297 A2, oder aus der WO 89/04819 A und können beispielsweise nach den dort beschriebenen Verfahren oder nach analogen Verfahren hergestellt werden. Die Verwendung der Verbindungen 1,1,1-Trifluor-7-phenyl-heptan-2-on und 5-(4-Methoxyphenyl)-1,1,1-trifluorpentan-2-on als Arzneimittel ist bislang noch nicht beschrieben worden. Gegenstand der Erfindung sind daher auch die Verbindungen 1,1,1-Trifluor-7-phenyl-heptan-2-on und 5-(4-Methoxyphenyl)-1,1,1-trifluorpentan-2-on zur Verwendung als Arzneimittel.

Die Verbindungen der Formel I können hergestellt werden, indem man
a) eine Verbindung der allgemeinen Formel XIa, 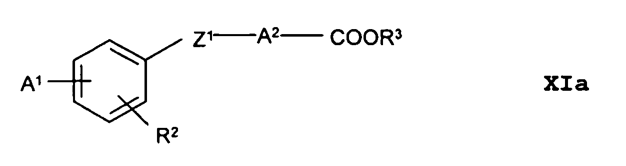 worin A¹, A² und R² obige Bedeutungen besitzen, R³ für C₁₋₄-Alkyl steht und Z¹ eine Bindung oder Sauerstoff bedeutet, mit Trifluormethyltrimethylsilan (= CF₃TMS) umsetzt, oder
b) eine Verbindung der allgemeinen Formel XIb" 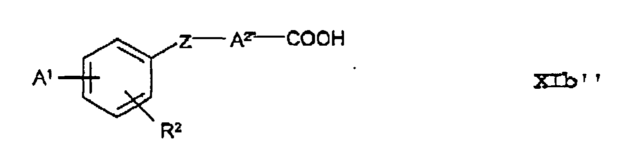 worin A¹, R² und Z obige Bedeutungen besitzen und A^{2"} C₁₋₃-Alkylen bedeutet, welches gegebenenfalls einfach durch C₁₋₁₂-Alkyl, C₁₋₁₂-Alkyl-phenyl oder C₁₋₁₂-Alkyl-oxyphenyl substituiert ist, mit einem Essigsäureanhydridderivat umsetzt und gegebenenfalls als Zwischenprodukte erhaltene En-Lactone mit Trifluormethyltrimethylsilan gemäß Verfahrensvariante a) umsetzt.

Die Umsetzung von Estern der Formel XIa mit CF₃TMS gemäß Verfahrensvariante a) kann auf an sich bekannte Weise, beispielsweise nach dem bei R. P. Singh et al. angegebenen oder hierzu analogen Verfahren erfolgen. Hierzu können Verbindungen der Formel XIa mit CF₃TMS, vorzugsweise in Anwesenheit eines Alkalimetallfluoridsalzes, insbesondere Cäsiumfluorid, und unter Ausschluß von wäßriger Feuchtigkeit in einem unter den Reaktionsbedingungen inerten organischen Lösungsmittel umgesetzt werden. Als Lösungsmittel eignen sich polare oder unpolare aprotische Lösungsmittel, beispielsweise Glykolether wie 1,2-Dimethoxyethan ("Glyme"). Das entstandene Zwischenprodukt kann anschließend in situ, beispielsweise durch Säurezugabe oder durch Zugabe von Fluoriden, insbesondere Tetrabutylammoniumfluorid (= TBAF), zu der gewünschten Verbindung der Formel I gespalten werden. Als Säuren zur Spaltung eignen sich Protonensäuren, beispielsweise Salzsäure. Die Umsetzung kann vorzugsweise bei Raumtemperatur (= RT) und unter Schutzgasatmosphäre durchgeführt werden.

Die Umsetzung von Carbonsäuren der Formel XIb'" mit einem Essigsäureanhydridderivat gemäß Verfahrensvariante b) kann auf an sich bekannte Weise, beispielsweise nach dem bei J. Boivin et al. angegebenen oder hierzu analogen Verfahren erfolgen. Hierzu können Verbindungen der Formel XIb" mit einem Essigsäureanhydridderivat wie Trifluoressigsäureanhydrid (= TFEA) oder Essigsäureanhydrid, vorzugsweise in Anwesenheit einer nicht nucleophilen organischen Base, beispielsweise einem organischen Amin oder Pyridin, in einem unter den Reaktionsbedingungen inerten organischen Lösungsmittel umgesetzt werden. Als Lösungsmittel eignen sich polare aprotische Lösungsmittel wie Halogenalkane, vorzugsweise Dichlormethan. Günstig ist es, die Reaktion unter Schutzgasatmosphäre und Ausschluß von wäßriger Feuchtigkeit durchzuführen. Die Reaktionstemperatur kann je nach eingesetztem Essigsäureanhydridderivat zwischen etwa -20 °C und 120 °C liegen. Sofern TFEA eingesetzt wird, kann die Reaktionstemperatur zwischen -20 °C und Raumtemperatur, vorzugsweise bei 0° bis 5 °C liegen. Sofern Essigsäureanhydrid eingesetzt wird, kann die Reaktionstemperatur zwischen 80 °C und 120 °C, vorzugsweise bei 90° bis 110 °C liegen. Das entstandene Zwischenprodukt kann anschließend durch Zugabe von Wasser, vorzugsweise Eiswasser, zu der gewünschten Verbindung der Formel I hydrolysiert werden. Sofern die Umsetzung von Carbonsäuren der Formel XIb", worin Z für Carbonyl steht, mit TFEA nicht auf direktem Weg zur Herstellung von Verbindungen der Formel I führt, können noch Zwischenprodukte wie ungesättigte Lactone der Carbonsäuren der Formel IIb entstehen. Diese Zwischenprodukte können auf an sich bekannte Weise, beispielsweise gemäß der vorstehend angegebenen verfahrensvariante a), in die gewünschten Verbindungen der Formel I verwandelt werden. In einer Ausführungsform zur Herstellung von Verbindungen der Formel If können Verbindungen der allgemeinen Formel IIa, 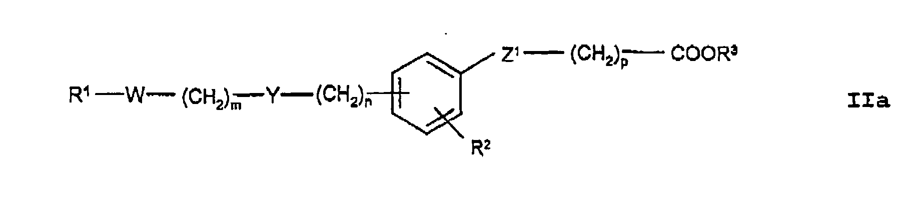 worin R¹, R², R³, W, Y, Z¹, m, n und p obige Bedeutungen besitzen, in der oben für die Verfahrensvariante a) angegebenen Weise analog zu den Verbindungen der Formel XIa eingesetzt werden, oder es können Verbindungen der allgemeinen Formel IIb, 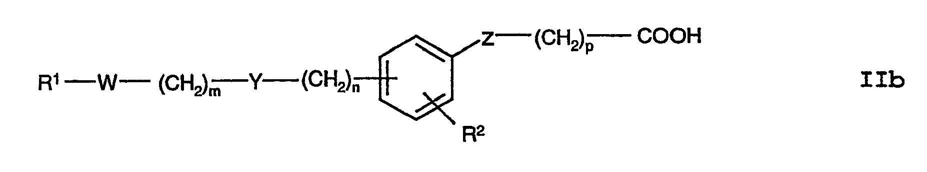 worin R¹, R², W, Y, Z, m, n und p obige Bedeutungen besitzen, in der oben für die Verfahrensvariante b) angegebenen Weise analog zu den Verbindungen der Formel XIb eingesetzt werden.

Die Ester der Formel XIa und die Säuren der Formel XIb sind an sich bekannt oder können vom Fachmann nach an sich bekannten Verfahren aus bekannten Ausgangsverbindungen hergestellt werden. So können beispielsweise Verbindungen der allgemeinen Formel XIc, 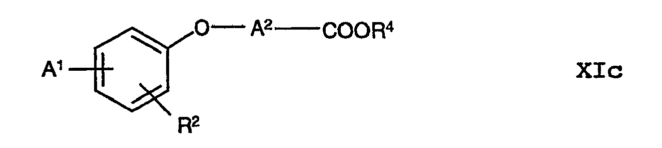 worin A¹, A², und R² obige Bedeutungen besitzen und R⁴ Wasserstoff oder Niederalkyl bedeutet, hergestellt werden, indem man eine Verbindung der allgemeinen Formel XII, 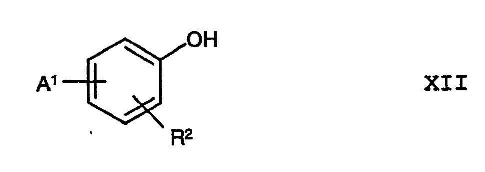 worin A¹ und R² obige Bedeutungen besitzen, mit einer Verbindung der allgemeinen Formel V,

X―A²―COOR³ V

worin A² und R³ obige Bedeutungen besitzen und X für eine Fluchtgruppe, insbesondere Halogen steht, umsetzt und nachfolgend gewünschtenfalls eine Niederalkylgruppe R³ auf an sich zur Esterspaltung bekannte Weise wieder abspaltet. Die Bromide der Formel V sind bevorzugt. Die Umsetzung kann auf für nucleophile Substitutionen an sich bekannte Weise durchgeführt werden. Beispielsweise kann man die Reaktion in einem unter den Reaktionsbedingungen inerten organischen Lösungsmittel wie einem dipolar aprotischen Lösungsmittel, vorzugsweise Tetrahydrofuran (= THF) oder Dimethylformamid (= DMF), und in Anwesenheit einer geeigneten nicht-nucleophilen organischen Base, vorzugsweise Kalium-tert.-butylat oder Natriumhydrid, durchführen. Üblicherweise wird bei Temperaturen zwischen etwa -40° und 80 °C gearbeitet. In einer speziellen Ausführungsform dieser Variante kann eine Verbindung der allgemeinen Formel IIe, 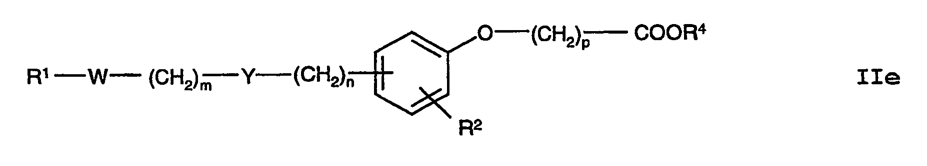 worin R¹, R², R⁴, W, Y, m, n und p obige Bedeutungen besitzen, hergestellt werden, indem man eine Verbindung der allgemeinen Formel IV 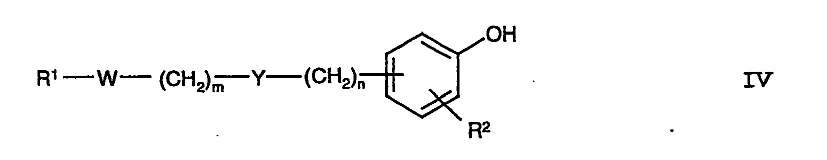 worin R¹, R², W, Y, m und n obige Bedeutungen besitzen, mit einer Verbindung der allgemeinen Formel Va,

X―(CH₂)ₚ―COOR³ Va

worin R³, p und X obige Bedeutungen besitzen, umsetzt und nachfolgend gewünschtenfalls eine Niederalkylgruppe R³ auf an sich zur Esterspaltung bekannte Weise wieder abspaltet.

Die Verbindungen der Formeln IV, V, Va und XII sind an sich bekannt oder können auf an sich bekannte Weise aus bekannten Verbindungen hergestellt werden.

Verbindungen der Formel XId, 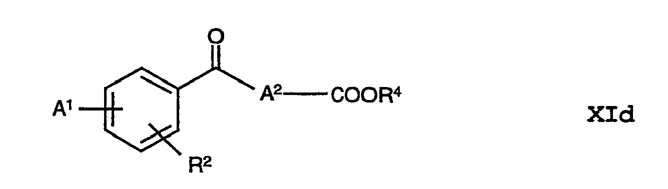 worin A¹, A², R², und R⁴ obige Bedeutungen besitzen, können beispielsweise hergestellt werden, indem man eine Verbindung der allgemeinen Formel XIII, 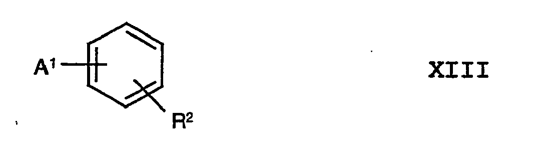 worin A¹ und R² obige Bedeutungen besitzen, mit einer Verbindung der allgemeinen Formel VII,

X³OC―A²―COOR³⁰¹ VII

worin A² obige Bedeutung besitzt, R³⁰¹ die oben für R³ angegebene Bedeutung besitzt oder gemeinsam mit X³ ein cyclisches Anhydrid bildet und X³ die vorstehend angegebene Bedeutung besitzt oder auch Halogen, insbesondere Chlor oder Brom bedeutet, umsetzt und nachfolgend gewünschtenfalls eine Niederalkylgruppe R³⁰¹ auf an sich zur Esterspaltung bekannte Weise wieder abspaltet. Die Umsetzung kann auf an sich bekannte Weise, beispielsweise unter den für Friedel-Crafts-Acylierungen bekannten Bedingungen in einem unter den Reaktionsbedingungen inerten organischen Lösungsmittel wie einem Halogenalkan, vorzugsweise Dichlormethan, und unter Katalyse durch eine Lewis-Säure wie Aluminiumtrichlorid durchgeführt werden. Verbindungen der Formel VII sind an sich bekannt oder können auf an sich bekannte Weise aus bekannten Ausgangsverbindungen hergestellt werden. Bevorzugte Verbindungen der Formel VII sind beispielsweise Glutarsäureanhydrid oder Glutarsäuremonoethylesterchlorid. Die Fälle, in denen Verbindungen der Formel XIII auf Grund ihres Substitutionsmusters als Ausgangsverbindungen zur Herstellung von Verbindungen der Formel XId durch Friedel-Crafts-Acylierung besonders geeignet sind, sind dem Fachmann geläufig. Verbindungen der Formel XIII sind an sich bekannt oder können auf an sich bekannte Weise aus bekannten Ausgangsverbindungen hergestellt werden. So können beispielsweise Verbindungen der Formel XIII, worin A¹ eine Gruppe der Formel R¹-W-A³-Y-(CH₂)ₙ- bedeutet und worin n nicht 0 bedeutet oder Y für eine Bindung steht, durch Wittig-Reaktion eines durch R² substituierten Benzaldehydderivates mit einem zur Einführung einer vorgenannten Gruppe A¹ geeigneten Phosphoniumsalzes oder Phosphonates in Anwesenheit einer Base und nachfolgende Hydrierung des intermediär erhaltenen Alkens erhalten werden.

Sofern in eine Verbindung der Formel XId, worin A² für unsubstituiertes C₁₋₂₀-Alkyl steht und R⁴ für Niederalkyl steht, eine C₁₋₁₂-Alkylgruppe, eine C₁₋₁₂-Alkyl-phenylgruppe oder eine C₁₋₁₂-Alkyl-oxyphenylgruppe eingeführt werden soll, kann dies auf an sich bekannte Weise durch nucleophile Substitutionsreaktion durchgeführt werden. Insbesondere kann eine vorgenannte Verbindung der Formel XId durch Umsetzung mit einer nicht-nucleophilen Base wie Natriumhydrid in einem unter den Reaktionsbedingungen inerten Lösungsmittel wie DMF deprotoniert und anschließend mit einem zur Einführung einer C₁₋₁₂-Alkylgruppe, einer C₁₋₁₂-Alkyl-phenylgruppe oder einer C₁₋₁₂-Alkyl-oxyphenylgruppe geeigneten reaktiven Reagenz alkyliert werden. Als reaktive Reagenzien zur Alkylierung eignen sich beispielsweise terminale Halogene, insbesondere terminale Bromide der vorgenannten Gruppen. Die Alkylsubstitution findet bei dieser Vorgehensweise üblicherweise an dem zu der Carbonylgruppe Z benachbarten aliphatischen Kohlenstoffatom der Verbindung der Formel XId statt.

In einer speziellen Ausführungsform der Herstellung von Verbindungen der Formel XId kann eine Verbindung der allgemeinen Formel IIf, 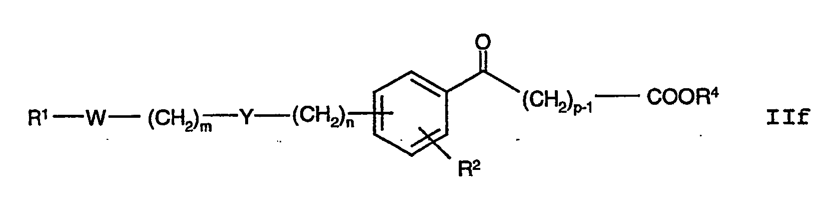 worin R¹, R², R⁴, W, Y, m, n und p obige Bedeutungen besitzen, hergestellt werden, indem man eine Verbindung der allgemeinen Formel VI, 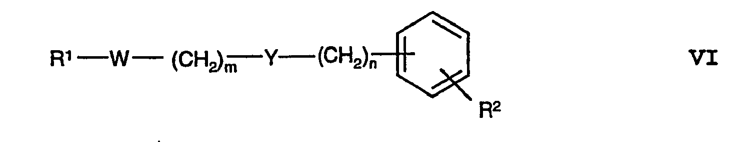 worin R¹, R², W, Y, m und n obige Bedeutungen besitzen, mit einer Verbindung der allgemeinen Formel VIIa,

X³OC―(CH₂)ₚ₋₁―COOR³⁰¹ VIIa

worin R³⁰¹, X³ und p obige Bedeutungen besitzen, in der vorstehend beschriebenen Weise umsetzt und nachfolgend gewünschtenfalls eine Niederalkylgruppe R³ auf an sich zur Esterspaltung bekannte Weise wieder abspaltet.

Verbindungen der Formel IIf können auch hergestellt werden, indem man eine Verbindung der allgemeinen Formel VIII, 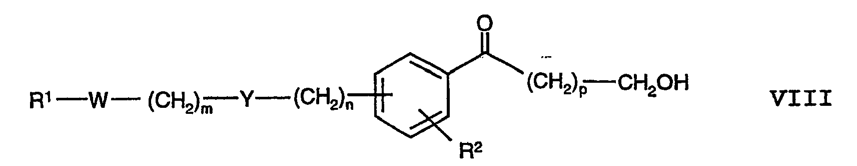 worin R¹, R², W, Y, m, n und p obige Bedeutungen besitzen, an der primären Alkoholfunktion oxidiert und die durch Oxidation entstandene Carboxygruppe gewünschtenfalls noch verestert. Die Oxidation kann auf an sich zur Überführung von primären Alkoholen in Carbonsäuren bekannte Weise, beispielsweise durch Umsetzung der Verbindungen der Formel VIII mit Chrom-VI-oxid, durchgeführt werden.

Verbindungen der Formel VIII können beispielsweise hergestellt werden, indem man eine Verbindung der allgemeinen Formel IX, 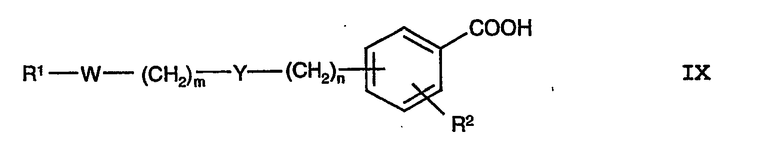 worin R¹, R², W, Y, m und n obige Bedeutungen besitzen, mit einer Verbindung der allgemeinen Formel X,

X¹―Mg―(CH₂)ₚ―CH₂OSG X

worin p obige Bedeutung besitzt, X¹ Halogen bedeutet und SG für eine abspaltbare Schutzgruppe steht, umsetzt und nachfolgend eine Schutzgruppe SG auf an sich bekannte Weise wieder abspaltet. Die Bromide und Jodide der Formel X sind bevorzugt. Die Umsetzung kann auf an sich zur Durchführung.von Grignard-Reaktionen bekannte Weise erfolgen. Insbesondere wird jeweils ein Äquivalent einer Verbindung der Formel IX mit zwei Äquivalenten einer Verbindung der Formel X umgesetzt. Geeignete Schutzgruppen SG sind bekannt, beispielsweise aus J. A. W. McOmie, "Protective Groups in Organic Chemistry", Plenum Press 1973 oder aus T. W. Green und P. G. Wuts "Protective Groups in Organic Synthesis", Wiley & Sons 1999. Der Fachmann kann jeweils geeignete Schutzgruppen durch Routinemethoden auswählen.

Die Verbindungen der Formel IX und die Verbindungen der Formel X sind an sich bekannt oder können auf an sich bekannte Weise aus bekannten Verbindungen hergestellt werden.

In einer Variante können Verbindungen der Formel XId hergestellt werden, indem man eine Verbindung der allgemeinen Formel XIV, 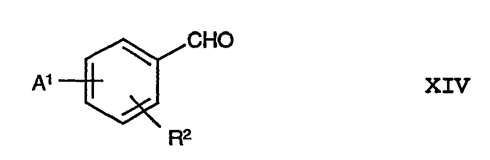 worin A¹ und R² obige Bedeutungen besitzen, mit einer Verbindung der Formel V umsetzt umsetzt und nachfolgend gewünschtenfalls eine Niederalkylgruppe R³ auf an sich zur Esterspaltung bekannte Weise wieder abspaltet. Die Umsetzung kann auf an sich bekannte Weise in einem unter den Reaktionsbedingungen inerten organischen Lösungsmittel wie einem cyclischen oder offenkettigen Diniederalkylether, vorzugsweise THF, durchgeführt werden. Insbesondere kann ein Aldehyd der Formel XIV in Anwesenheit von katalytisch wirkenden Reagenzien wie TBAF und Trimethylsilylcyanid zuerst bei einer Temperatur zwischen -100 °C und -60 °C in ein silyliertes Cyanhydrin-Zwischenprodukt überführt werden, welches nach Deprotonierung durch eine nicht-nucleophile Base, vorzugsweise eine organische Base wie Lithium-bis(trimethylsilyl)amid oder Lithiumdiisopropylamid (= LDA) und bei erhöhter Temperatur, vorzugsweise RT, anschließend mit einer Verbindung der Formel V umgesetzt werden kann. Verbindungen der Formel XIV sind an sich bekannt oder können auf an sich bekannte Weise aus bekannten Ausgangsverbindungen hergestellt werden.

In einer weiteren Variante können Verbindungen der Formel XId hergestellt werden, indem man eine Verbindung der allgemeinen Formel XV, 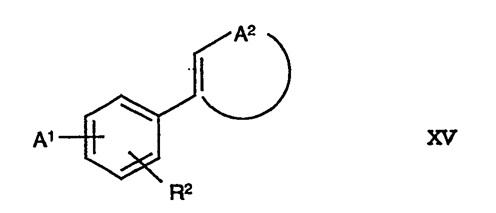 worin A¹, A² und R² obige Bedeutungen besitzen, mit einem geeigneten Oxidationsmittel oxidiert. Die Oxidation kann auf an sich bekannte Weise in einem unter den Reaktionsbedingungen inerten organischen Lösungsmittel wie einem aromatischem Lösungsmittel, insbesondere Toluol, bei Temperaturen zwischen -20 °C und RT durchgeführt werden. Als Oxidationsmittel eignet sich beispielsweise Kaliumpermanganat, vorzugsweise in Gegenwart eines Phasentransferkatalysators wie Aliquat® 336. Ebenso kann die Oxidation durch Ozonolyse mit anschließender Aufarbeitung in Anwesenheit eines Oxidationsmittels ausgeführt werden.

Verbindungen der Formel XV können hergestellt werden, indem man eine Verbindung der allgemeinen Formel XVII, 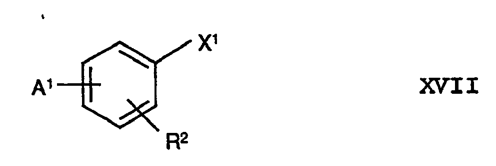 worin A¹, R² und X¹ obige Bedeutungen besitzen, mit einem cyclischen Keton der allgemeinen Formel XVIII, 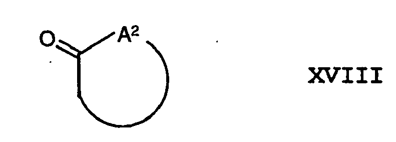 worin A² obige Bedeutung besitzt, auf an sich bekannte Weise unter den Bedingungen einer Grignard-Reaktion umsetzt, und das entstandene Zwischenprodukt durch säurekatalysierte Wasserabspaltung anschließend zu einer Verbindung der Formel XV umsetzt.

Verbindungen der allgemeinen Formel XIe, 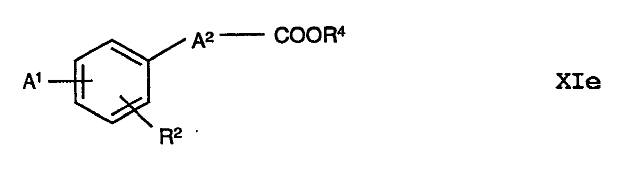 worin A¹, A², R² und R⁴ obige Bedeutungen besitzen, können beispielsweise hergestellt werden, indem man in einer Verbindung der Formel XId auf an sich bekannte Weise eine Carbonylgruppe Z selektiv hydriert. Die Hydrierung kann in einem unter den Reaktionsbedingungen inerten organischen Lösungsmittel wie Essigsäureethylester (= EE) als heterogen katalysierte Hydrierung durchgeführt werden. Als Katalysatoren eignen sich z. B. heterogene Edelmetallkatalysatoren wie Palladium auf Aktivkohle. In einer speziellen Ausführungsform dieser Variante zur Herstellung von Verbindungen der Formel XIa kann eine Verbindung der allgemeinen Formel IIg, 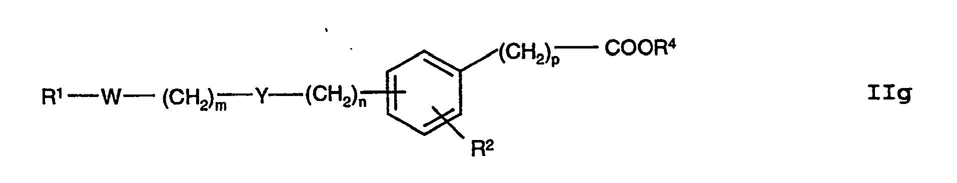 worin R¹, R², R⁴, W, Y, m, n und p obige Bedeutungen besitzen, hergestellt werden, indem man eine entsprechende Verbindungen der Formel IIf selektiv hydriert.

Zur Herstellung von Verbindungen der Formel I, worin A¹ für eine Gruppe der Formel R¹-W-A³-Y-(CH₂)ₙ- steht, kann die Gruppe A¹ gewünschtenfalls auf verschiedenen Wegen in jeweils geeignete Vorläuferverbindungen der Verbindungen der Formel I eingeführt werden. Hierfür eignen sich je nach Struktur der einzuführenden Gruppe R¹-W-A³-Y-(CH₂)ₙ- an sich bekannte Reaktionen.

Zur Herstellung von Verbindungen der Formel I, worin Y Sauerstoff bedeutet, kann die Gruppe der Formel R¹-W-A³-Y-(CH₂)ₙ- beispielsweise aufgebaut werden, indem man eine Gruppe der Formel R¹-W-A³- in Form eines zu einer Kopplung geeineten reaktiven Derivates wie eines Halogenderivates mit einer geeigneten Vorläuferverbindung der Formel I koppelt, welche in dem die Gruppe R² tragenden Phenylring zusätzlich durch HO-(CH₂)ₙ- substituiert ist. Die Vorläuferverbindungen der Formel I, welche in dem die Gruppe R² tragenden Phenylring zusätzlich durch HO-(CH₂)ₙ- substituiert sind, können dabei selbst Verbindungen der Formel I darstellen. Die Kopplung kann im Sinne einer Etherbildung, beispielsweise durch nucleophile Substitutionsreaktion, durchgeführt werden. In einer beispielhaften Ausführungsform der vorgenannten Etherbildung kann eine Verbindung der allgemeinen Formel IIc 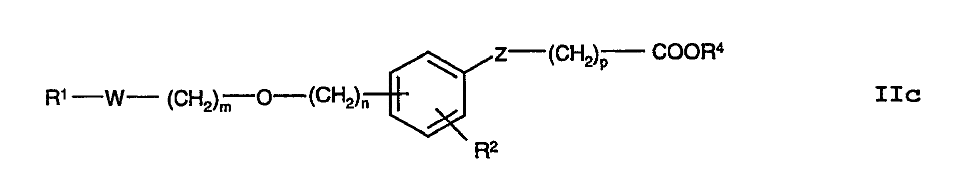 worin R¹, R², R⁴, W, Z, m, n und p obige Bedeutungen besitzen, hergestellt werden, indem man eine Verbindung der allgemeinen Formel IId 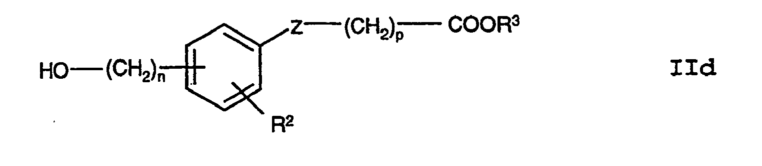 worin R², R³, Z, n und p obige Bedeutungen besitzen, mit einer Verbindung der allgemeinen Formel III,

R¹―W― (CH₂)ₘ―X III

worin R¹, W, X und m obige Bedeutungen besitzen, umsetzt und eine Niederalkylgruppe R³ nachfolgend gewünschtenfalls wieder abspaltet.

Zur Herstellung von Verbindungen der Formel I kann die Gruppe der Formel R¹-W-A³-Y-(CH₂)ₙ- beispielsweise eingeführt werden, indem man die Gruppe der Formel R¹-W-A³-Y-(CH₂)ₙ- in Form eines zu einer Kopplung geeineten reaktiven Derivates wie eines Boronsäurederivates, mit einer geeigneten Vorläuferverbindung der Formel I, welche in dem die Gruppe R² tragenden Phenylring durch eine geeignete Abgangsgruppe wie Halogen oder die Trifluormethansulfonylgruppe substituiert ist, durch Edelmetall-, vorzugsweise Pd(0)-, katalysierte Reaktion koppelt. Derartige insbesondere Pd(0)- katalysierte, Kopplungsreaktionen sind an sich bekannt, beispielsweise in Gestalt der Heck-, Stille- oder der Suzuki-Kopplungen. Zur Durchführung der Pd(0)-katalysierten Kopplungsreaktionen geeignete Vorläuferverbindungen der Formel I und reaktive Derivate der Gruppe der Formel R¹-W-A³-Y-(CH₂)- sind dem Fachmann bekannt oder können durch Routinemethoden aufgefunden werden. Die Vorläuferverbindungen der Formel I, welche in dem die Gruppe R² tragenden Phenylring durch Halogen substituiert sind, können dabei selbst Verbindungen der Formel I darstellen.

Weitere Verbindungen der Formeln XIa und XIb können entsprechend den vorstehend beschriebenen Herstellungsverfahren oder analog zu diesen Herstellungsverfahren auf an sich bekannte Weise hergestellt werden.

Ferner können Verbindungen der Formel I analog zu in der WO 99/15129 oder in der EP 0 434 297 A2 beschriebenen Verfahren hergestellt werden.

Die Verbindungen der Formel I können auf an sich bekannte Weise aus dem Reaktionsgemisch_isoliert und gereinigt werden.

Die vorliegende Erfindung umfaßt neben den eine Trifluoracetylgruppe enthaltenden freien Verbindungen der Formel I auch an der Trifluoracetylgruppe solvatisierte, insbesondere hydratisierte, Verbindungen der Formel I.

Die erfindungsgemäßen Verbindungen der Formel I eignen sich zur Hemmung der Lipase, insbesondere zur gegebenenfalls selektiven Hemmung der Pankreaslipase von größeren Säugetieren, insbesondere Menschen. Verbindungen mit lipasehemmenden Eigenschaften sind in der Lage, sofern sie vorzugsweise gemeinsam mit fetthaltiger Nahrung dem Verdauungstrakt zugeführt werden, den Anteil der vom Körper tatsächlich verdauten Nahrungsfette an den insgesamt aufgenommenen Nahrungsfetten herabzusetzen. Auf diese Weise kann die Fettresorption bei Säugetieren, insbesondere Menschen, herabgesetzt werden. Die erfindungsgemäße Gruppe von Verbindungen erscheint somit geeignet zur Behandlung und/oder Prophylaxe von Obesitas sowie von damit einhergehenden Begleiterkrankungen und/oder Folgeerkrankungen. Zu den begleitenden Erkrankungen der Obesitas oder deren Folgeerkrankungen, welche jeweils mit den erfindungsgemäßen Verbindungen therapierbar sein können, zählt das Metabolische Syndrom Unter dem Begriff "Metabolisches Syndrom" wird üblicherweise ein Komplex von Krankheitsbildern zusammengefaßt, welcher hauptsächlich Bluthochdruck (= Hypertonie), insbesondere arteriellen Bluthochdruck, Insulinresistenz, insbesondere Diabetes mellitus Typ II, Dyslipoproteinämie, insbesondere als Hypertriglyceridämie, einhergehend mit erniedrigtem HDL-Cholesterin auftretende Dyslipoproteinämie, sowie Hyperurikämie, welche zur Gicht führen kann, umfaßt.

Die lipasehemmenden Eigenschaften der Verbindungen der Formel I lassen sich z. B. durch einen in vitro-Aktivitätstest belegen. In diesem Test wird die Hemmung der lipolytischen Wirkung von Schweinepankreaslipase gegenüber dem Testsubstrat p-Nitrophenylpalmitat unter dem Einfluß der Testsubstanzen der Formel I bestimmt. Dabei wird die Änderung der relativen Extinktion der Untersuchungslösungen gemessen, welche durch die lipolytische Freisetzung von p-Nitrophenol aus p-Nitrophenylpalmitat verursacht wird. Angegeben wird die nach Zugabe der Testsubstanzen verbliebene Restaktivität der Lipase in Prozent, bezogen auf die ursprüngliche lipolytische Ausgangsaktivität. Die angegebenen Beispielsnummern beziehen sich auf die nachfolgend angegebenen Herstellungsbeispiele.

Es werden die nachfolgend angegebenen Reagenzien hergestellt:

### 1. Substratlösung

Zur Herstellung einer "Lösung A" werden 45 mg p-Nitrophenylpalmitat in 15 ml Isopropanol durch Beschallen mit Ultraschall gelöst. Zur Herstellung einer "Lösung B" werden 310 mg Na-Desoxycholat-Trockensubstanz und 150 mg Gummi arabisch in 135 ml 0,05 M Natriumphosphatpuffer (pH = 8,0) gelöst. 5 Minuten vor Testdurchführung werden 9 ml "Lösung B" mit 1 ml "Lösung A" zu einer "Lösung C" gemischt und auf 38 °C temperiert.

### 2. Pankreaslipaselösung

100 mg FIP-Lipase-Standard LS7 (Schweinepankreaslipase, 36.700 FIP-E/g) werden in 50 ml eiskalter 1%iger wäßriger Natriumchloridlösung gelöst und durch ein 0,2 µm-Spritzenfilter filtriert. Nach Bestimmung der Lipaseaktivität gemäß FIP wird die Lösung mit 1%iger wäßriger Natriumchloridlösung auf eine Aktivität von 40 FIP-E/ml eingestellt. Von der Pankreaslipaselösung werden für eine Eichreihe durch Verdünnen mit 1%iger wäßriger Natriumchloridlösung auch verdünnte Pankreaslipaselösungen hergestellt, welche Aktivitäten von jeweils 10, 20 und

30 FIP-E/ml aufweisen.

### 3. Inhibitorlösungen

Die Lipase-inhibitorisch wirksamen Verbindungen der Formel I werden in verschiedenen Konzentrationen in Dimethylsulfoxid (= DMSO) gelöst, so daß in 100 µl Inhibitorlösung zwischen 2,0 und 800 nmol des Inhibitors vorhanden sind. Zur Bestimmung des Leerwertes wird reines

DMSO verwendet.

### Testdurchführung

100 µl der wie vorstehend beschrieben hergestellten Inhibitorlösung einer bestimmten Konzentration werden 5 Minuten lang im Küvettenwechsler eines Photometers (Biochrome 4.060) temperiert. Anschließend gibt man 1 ml der obenbezeichneten "Lösung C" unter Rühren zu. Durch Zugabe von 100 µl Pankreaslipaselösung unter Rühren wird dann die Reaktion gestartet. 2 Minuten nach dem Reaktionsstart wird die Extinktion jeder Probe bei 405 nm 4 Minuten lang erfaßt.

Zur Bestimmung der Lipaseaktivität wird jeweils neben der Probenmessung auch eine Eichreihe vermessen. Die Eichproben enthalten dabei keinen Inhibitor, aber Pankreaslipaselösung unterschiedlicher Aktivität (10, 20, 30 und 40 FIP-E/ml, s. o.). Die Eichreihe dient zur Bestimmung der Lipaseaktivität. Zur Ermittlung der Eichreihe trägt man die eingesetzten Lipaseeinheiten gegen die jeweils ermittelten Extinktionswerte (dE/min) auf. Anhand dieser Eichgeraden und der für die jeweiligen Inkubationsansätze ermittelten Extinktionswerte läßt sich für jede Probe die Lipaseaktivität bestimmen. Entsprechend der eingesetzten Lipaseaktivität kann die Restaktivität und somit die inhibitorische Wirkung der Testsubstanzen berechnet werden. Zur Bestimmung des Leerwertes werden 100 µl DMSO mit 1 ml "Lösung C" gemischt und die Reaktion wird durch Zugabe von 100 µl einer Lipaselösung gestartet.

### Testauswertung

Der Meßwert des Photometers pro Ansatz (dE/min.) ergibt sich aus der Differenz der Extinktionswerte nach der zweiten und der sechsten Minute (dE) dividiert durch die Meßdauer (dt = 4 min.). Analog wird der Leerwert ermittelt und anschließend vom Meßwert subtrahiert. Mittels des auf diese Weise errechneten dE-Wertes kann die Restaktivität über die Eichgerade abgelesen werden.

In dem vorstehend angegebenen Pankreaslipase-Aktivitätstest bewirkten die nachstehend angegebenen Testsubstanzen jeweils in einer Konzentration (= c) von 0,727 µM (sofern nicht anders angegeben) eine Hemmung der Lipaseaktivität auf den nachfolgend angegebenen Bruchteil der ursprünglichen Ausgangsaktivität. Die Verbindungen der Beispiele 17, 28, 29, 32, 39, 40, 47, 48, 56, 58 und 59 wurden in einer Konzentration von jeweils 0,364 µM gemessen. Die Verbindungen der Beispiele 21, 35, 37, 38, 49, 50, 51, 54, 55 und 64 wurden in einer Konzentration von jeweils 0,182 µM gemessen. Die Verbindungen der Beispiele 46, 61, 62 und 65 wurden in einer Konzentration von jeweils 0,091 µM gemessen. Die angegebenen Beispielsnummern beziehen sich auf die nachfolgend angegebenen Herstellungsbeispiele.

Die Verbindungen der Beispiele 1-14, 16-23, 25-29, 31-32, 37-41, 44-51 54-59 und 61-62 bewirkten eine Hemmung der Lipaseaktivität auf höchstens 60 % ihrer ursprünglichen Ausgangsaktivität.

Die Verbindungen der Beispiele 1-4, 6, 9-10, 16-19, 21-23, 26-28, 32, 35, 37-41, 44, 45, 47, 49, 51, 54, 55, 57 und 58 bewirkten eine Hemmung der Lipaseaktivität auf höchstens 35 % ihrer ursprünglichen Ausgangsaktivität.

Die Verbindungen der Beispiele 4, 32, 34, 37, 38, 40, 41, 44, 47, 51, 54, 55, 57-62 und 65 bewirkten eine Hemmung der Lipaseaktivität auf höchstens 20 % ihrer ursprünglichen Ausgangsaktivität.

Die Verbindungen der Formel I können in üblichen pharmazeutischen Zubereitungen verabreicht werden. Die zu verwendenden Dosen können individuell verschieden sein und variieren naturgemäß je nach Art des zu behandelnden Zustandes und der verwendeten Substanz. Im allgemeinen eignen sich zur Applikation am Menschen und an größeren Säugetieren jedoch Arzneiformen mit einem Wirkstoffgehalt von 10 bis 500 mg, insbesondere 50 bis 250 mg Wirkstoff pro Einzeldosis.

Die Verbindungen können erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt. Diese Präparate können pharmazeutisch übliche anorganische und/oder organische Trägerstoffe, wie z. B. Talkum, Milchzucker oder Stärke, neben pharmazeutisch üblichen Hilfsmitteln, beispielsweise Gleitmitteln oder Tablettensprengmitteln, enthalten. Flüssige Präparate wie Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z. B. Konservierungsmittel, Geschmackskorrigenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden.

Die nachfolgende angegebenen Herstellungsbeispiele zur Herstellung von Verbindungen der allgemeinen Formel I sollen die Erfindung näher beschreiben

### Beispiel 1:

### 5-{4-[(Benzyloxy)-methyl]-phenoxy}-1,1,1-trifluorpentan-2-on

A) 100 g 4-Hydroxybenzylalkohol wurden in 600 ml getrocknetem DMF gelöst und mit 213,2 g gemahlenem und getrocknetem Kaliumcarbonat versetzt. Zu dieser Vorlage gab man unter Feuchtigkeitsausschluß 126 ml 4-Brombuttersäureethylester. Anschließend wurde die entstandene Reaktionsmischung 18 Stunden (= h) lang bei Raumtemperatur und 4 h lang bei 45 °C gerührt. Nach Abkühlung auf Raumtemperatur wurde mit Methyl-tert.-butylether (= MTBE) verdünnt und der Niederschlag abgesaugt. Das Filtrat wurde unter vermindertem Druck eingeengt und der Rückstand wurde mit einem 1:1-Gemisch aus EE und MTBE aufgenommen. Die erhaltene Lösung wurde dann mit Eiswasser, verdünnter wäßriger Natronlauge und gesättigter Kochsalz-Lösung gewaschen. Die organische Phase wurde nach Trocknung über Natriumsulfat im Vakuum eingedampft und der erhaltene Rückstand wurde aus 300 ml eines Lösemittelgemisches aus 95 % n-Hexan und 5 % MTBE kristallisiert. Die erhaltenen Kristalle wurden von der Mutterlauge durch Vakuumfiltration getrennt und im Vakuum bei 30 °C getrocknet. Man erhielt insgesamt 154,7 g 4-[4-(Hydroxymethyl)-phenoxy]-buttersäureethylester, Schmelzpunkt (= Fp.) = 34-36 °C.
B) Unter Schutzgasatmosphäre wurden 11,9 g des vorstehend erhaltenen Produkts in 120 ml trockenem Tetrahydrofuran (= THF) gelöst und auf -30 °C abgekühlt. Dazu ließ man eine Lösung von 5,7 g Kalium-tert.-butylat in 50 ml trockenem THF zutropfen. Nach einstündigem Rühren wurden bei dieser Temperatur eine Lösung von 6,1 ml Benzylbromid in 10 ml trockenem THF hinzugegeben. Man rührte 1 Stunde lang bei -30 °C, ließ auf 0 °C kommen und verdünnte dann mit 150 ml MTBE. Die organische Phase wurde nach Waschen mit Kaliumhydrogensulfat-Lösung und Wasser über Natriumsulfat getrocknet und unter vermindertem Druck eingedampft. Der erhaltene Rückstand wurde an Kieselgel mit einem Laufmittelgemisch aus 4 Teilen n-Hexan und einem Teil EE gereinigt. Eindampfen und Trocknen der Produktfraktionen im Vakuum lieferten 5,8 g 4-{4-[(Benzyloxy)-methyl]-phenoxy}-buttersäureethylester, IR(Film): 2937, 2859, 1733, 1612, 1513, 1247 cm⁻¹.
C) Unter Schutzgasatmosphäre wurden 5,6 g des vorstehend erhaltenen Produkts in 25 ml 1,2-Dimethoxyethan ("Glyme"), das zuvor über aktiviertem Molsieb getrocknet worden war, gelöst. Dazu gab man unter Rühren zuerst 2,55 ml Trifluormethyl-trimethylsilan und dann 20 mg getrocknetes Cäsiumfluorid schnell hinzu. Das entstandene Reaktionsgemisch ließ man 2 h lang bei RT rühren. Zu dieser Lösung wurde dann 5,4 g TBAF hinzugefügt und es wurde weitere 2 h lang bei RT gerührt. Das Reaktionsgemisch wurde dann mit 50 ml MTBE verdünnt und mit Wasser gewaschen. Die organische Phase wurde über Natriumsulfat getrocknet und unter vermindertem Druck eingedampft. Der verbliebene Rückstand wurde an 200 g Kieselgel mit einem Laufmittelgemisch aus n-Hexan und Essigester 4:1 flashchromatographiert. Nach Eindampfen der Produktfraktionen und Trocknung im Vakuum bei 60 °C erhielt man 4,2 g der Titelverbindung als Öl, IR (Film): 3033, 2940, 2861, 1764, 1612, 1587, 1513, 1208 cm⁻¹; Massenspektum (= MS) m/z: 352, 261, 107, 91

### Beispiel 2:

### 1,1,1-Trifluor-6-(4-methoxyphenyl)-hexan-2-on

A) Zu einer Lösung von 11,4 g Glutarsäureanhydrid in 250 ml Dichlormethan gab man 16,3 ml Anisol. Unter Eiskühlung wurden dann portionsweise 26,7 g wasserfreies Aluminiumtrichlorid hinzugegeben. Das rötlich gefärbte Reaktionsgemisch wurde 3 h lang bei 0 °C gerührt und anschlie-ßend auf ein Gemisch aus Eis und verdünnter Salzsäure gegossen. Der dabei entstandene weiße Niederschlag wurde auf unter Vakuum abgesaugt, mit Wasser und etwas Dichlormethan gewaschen und schließlich unter vermindertem Druck bei 60 °C getrocknet. Man erhielt 13,6 g 5-(4-Methoxyphenyl)-5-oxo-valeriansäure, Fp. = 139-142 °C.
B) 4,4 g des vorstehend erhaltenen Produkts wurden in einem Lösungsmittelgemisch, bestehend aus 200 ml EE und 50 ml Eisessig, gelöst. Nach Zugabe von 0,16 g Palladium-Katalysator (10 % auf Kohle) wurde bei 2,5 bar Wasserstoffdruck und 50 °C acht h lang hydriert. Nach Abfiltration des Katalysators und Nachwaschen mit EE wurde unter vermindertem Druck eingedampft und das Rohprodukt zur weiteren Reinigung über eine Kieselgelsäule filtriert (Laufmittel: Dichlormethan/Methanol 9:1). Nach Eindampfen im Vakuum erhielt man 4,2 g 5-(4-Methoxyphenyl)-valeriansäure, Fp. = 107-111 °C.
C) Zu einer Lösung von 8,2 ml Trifluoressigsäureanhydrid in 10 ml trockenem Dichlormethan wurde unter Schutzgasatmosphäre und Eiskühlung eine Lösung von 2,0 g des vorstehend erhaltenen Produkts in 20 ml trockenem Dichlormethan hinzugetropft, wobei die Temperatur 5 °C nicht überstieg. Nach Kühlung auf 0 °C wurden 7,15 ml Pyridin hinzugetropft. Das Reaktionsgemisch wurde anschließend eine Stunde lang bei 0 °C und zwei h bei RT gerührt. Danach wurden unter Kühlung 80 ml Eiswasser langsam hinzugegeben und 30 Minuten gerührt. Es wurde mit Wasser versetzt und die wäßrige Phase wurde mit Dichlormethan extrahiert. Die organische Phase wurde dann mit gesättigter Natriumchloridlösung gewaschen und über Natriumsulfat getrocknet. Anschließend wurde die organische Phase unter vermindertem Druck eingedampft. Der erhaltene Rückstand wurde an Kieselgel flashchromatographiert (Laufmittel: zunächst n-Hexan, welches kontinuierlich durch EE ersetzt wurde). Eindampfen der Produkt-Fraktionen ergab 1,1 g der Titelverbindung als Öl, IR (Film): 2937, 2860, 1764, 1613, 1584, 1513, 1209, 827, 709 cm⁻¹; MS m/z: 260, 191, 147, 121, 91.

### Beispiel 3:

### 5-[4-(Benzyloxy)phenoxy]-1,1,1-trifluorpentan-2-on

A) Zu einer Suspension von 25 g 4-Benzyloxyphenol und 25,7 g gemahlenem und getrocknetem Kaliumcarbonat in 125 ml trockenem DMF wurden 27,2 ml 4-Brombuttersäureethylester hinzugegeben. Anschließend wurde das Reaktionsgemisch 40 h lang bei RT unter Schutzgasatmosphäre gerührt. Man filtrierte vom Feststoff ab und wusch mit MTBE nach. Das Filtrat wurde dann unter vermindertem Druck eingeengt und der verbliebene Rückstand wurde mit MTBE aufgenommen. Die organische Phase wurde der Reihe nach mit Wasser und wäßriger Kochsalzlösung gewaschen. Anschließend wurde die organische Phase über Natriumsulfat getrocknet und im Vakuum eingedampft, wobei das Rohprodukt kristallisierte. Umkristallisation aus EE/n-Hexan unter Eiskühlung ergaben 35,8 g 4-[4-(Benzyloxy)-phenoxy]-buttersäureethylester, Fp.= 51-52 °C.
B) Unter Schutzgasatmosphäre wurden 24,8 g des vorstehend erhaltenen Produkts in 115 ml trockenem Glyme gelöst, mit 13,2 ml Trifluormethyltrimethylsilan und 126 mg Cäsiumfluorid versetzt und anschließend 2 h lang bei RT gerührt. Zu diesem Reaktionsgemisch wurden dann 25 g TBAF zugegeben und es wurde weitere 3 h lang gerührt. Anschließend wurde mit MTBE verdünnt und die organische Phase wurde mit Eiswasser und Kochsalzlösung gewaschen. Dann wurde die organische Phase über Natriumsulfat getrocknet und unter vermindertem Druck eingedampft, wobei sich ein kristalliner Rückstand bildete. Dieser Rückstand wurde aus EE/n-Hexan umkristallisiert. Man erhielt 25,6 g der Titelverbindung, Fp. = 51-53 °C.

### Beispiel 4:

### 6,6,6-Trifluor-1-(4-methoxyphenyl)hexan-1,5-dion

A) Unter Schutzgastmosphäre wurde zu 51,8 ml Trifluoressigsäureanhydrid, gelöst in 300 ml trockenem Dichlormethan, eine Lösung von 20,7 g 5-(4-Methoxyphenyl)-5-oxo-valeriansäure (Herstellung s. Beispiel 2A)) in 200 ml trockenem Dichlormethan unter Eiskühlung langsam zugetropft, so daß die Temperatur zwischen 0 und 5 °C lag. Unter denselben Bedingungen wurden anschließend noch 45,1 ml Pyridin zugetropft. Dann wurde das Reaktionsgemisch eine Stunde lang bei 0 °C und zwei h lang bei RT gerührt. Man goß das Reaktionsgemisch auf Eis und wusch die organische Phase nacheinander mit Wasser und gesättigter Kochsalzlösung. Nach Trocknung über Natriumsulfat wurde im Vakuum eingedampft. Flashchromatographie des erhaltenen Rückstandes an Kieselgel mit n-Hexan, dem ein stetig erhöhter Anteil an EE zugefügt wurde, lieferte 16,5 g 6-(4-Methoxyphenyl)-3,4-dihydro-2H-pyran-2-on, Fp. = 77,4-79,8 °C.
B) Unter einer Stickstoff-Atmosphäre wurden 16,3 g des vorstehend erhaltenen Produktes in 100 ml trockenem Glyme gelöst und anschließend mit Molekularsieb versetzt. Unter Rühren wurde über eine Dauer von 30 Minuten insgesamt 12,3 ml Trifluormethyltrimethylsilan hinzugegeben. Nach Zugabe einer Spatelspitze Cäsiumfluorid wurde 2 h bei RT gerührt. Dann wurden 25,18 g TBAF-trihydrat hinzugegeben und weitere 30 Minuten lang gerührt. Man nahm das Reaktionsgemisch mit MTBE auf und wusch die organische Phase der Reihe nach mit Wasser und gesättigter Kochsalzlösung. Die organische Phase wurde abgetrennt, über Natriumsulfat getrocknet und im Vakuum eingeengt. Der erhaltene Rückstand wurde mit einem Gemisch aus n-Hexan/ EE (4:1), dem ein stetig erhöhter Anteil an EE hinzugefügt wurde, an Kieselgel flashchromatographiert. Nach Eindampfen der Produktfraktionen erhielt man 12,2 g der Titelverbindung, Fp. = 67,5-68,9 °C.

Nach den vorstehend angegebenen Verfahren oder analog zu diesen Verfahren können auch die nachstehend in Tabelle 1 angegebenen Verbindungen der Formel I hergestellt werden.

**Tabelle 1:**

| Weitere Verbindungen der Formel I | | | | | | | | | | |
|---|---|---|---|---|---|---|---|---|---|---|
| **Bsp** | **A**¹ | | | | | | **R**^{**2**} | **Z** | **A**^{**2**} | **Fp. [°C]; Ms m/z; IR [cm**^{**-1**}] |
| | **Pos A**^{**1**} | **R**^{**1**} | **W** | **A**^{**3**} | **y** | **n** | | | | |
| **5** | 4- | H | B | B | B | O | H | O | -(CH₂)₁₀- | 47 - 49°C |
| **6** | 4- | H | B | B | B | O | H | B | -(CH₂)₇- | m/z: 272,104,91 |
| **7** | 4- | H | B | B | B | O | H | B | -(CH₂)₄- | m/z: 230, 117, 91 |
| **8** | 4- | H | B | B | B | O | H | B | -(CH₂)₉- | m/z: 300, 133, 117, 91 |
| **9** | 4- | H | B | B | B | O | H | B | -(CH₂)₅- | m/z:244,117,91 |
| **10** | 4- | H | B | B | B | O | H | B | -(CH₂)₆- | m/z: 258, 117, 104, 91 |
| **11** | 4- | -CH₃ | B | B | O | O | H | B | -(CH₂)₃- | m/z: 246, 134, 121, 91 |
| **12** | 4- | -C₆H₅ | B | -CH₂- | O | O | H | B | -(CH₂)₃- | 63 - 65°C |
| **13** | 4- | H | B | B | B | O | H | C(O) | -(CH₂)₄- | 59 - 61°C |
| **14** | 4- | -C₆H₅ | B | -CH₂- | O | O | H | B | -(CH₂)₂- | 66-69°C |
| **15** | 4- | -CH₃ | B | B | B | 0 | H | O | -(CH₂)₃- | 2927, 1764, 1614, 1513 cm⁻¹ |
| **16** | 4- | -C₆H₅ | B | -(CH₂)₃- | O | 1 | H | O | -(CH₂)₃- | m/z: 380,119,91 |
| **17** | 4- | 2-Naphthyl | B | -(CH₂)₂- | O | 1 | H | O | -(CH₂)₃- | 87 - 82°C |
| **18** | 4- | -CH₃ | B | -(CH₂)₇- | O | 1 | H | O | -(CH₂)₃- | m/z: 374, 245, 139, 107 |
| **19** | 4- | -C₆H₅ | B | -(CH₂)₄- | O | 1 | H | O | -(CH₂)₃- | m/z: 394,245,139,131,107 |
| **20** | 4- | c-C₆H₁₁ | B | -(CH₂)₂- | O | 1 | H | O | -(CH₂)₃- | m/z: 372, 245, 139, 107 |
| **21** | 4- | -C₆H₅ | O | -(CH₂)₇- | O | 1 | H | O | -(CH₂)₃- | 42 - 44 °C |
| **22** | 4- | -C₆H₅ | B | -(CH₂)₃- | O | O | H | B | -(CH₂)₄- | 60 - 63 °C |
| **23** | 4- | -C₂H₅ | 0 | -(CH₂)₃- | O | 1 | H | O | -(CH₂)₃- | m/z: 396,261,139,123,107 |
| **24** | 4- | -C₂H₅ | O | -(CH₂)₃- | O | 1 | H | O | -(CH₂)₃- | m/z: 348,261,123,107 |
| **25** | 4- | -CH₃ | B | -(CH₂)₁₀- | O | 1 | H | 0 | -(CH₂)₃- | 2927,1765,1613,1513 cm⁻¹ |
| **26** | 4- | -CH(CH₃)₂ | B | -(CH₂)₅- | O | 1 | H | O | -(CH₂)₃₋ | m/z: 332, 245, 139, 107 |
| **27** | 4- | -C₆H₅ | O | -(CH₂)₅- | O | 1 | H | O | -(CH₂)₃- | m/z: 438, 347, 261,123,107, 91 |
| **28** | 4- | c-C₆H₁₁ | B | -(CH₂)₄- | O | 1 | H | O | -(CH₂)₃- | m/z: 400, 245,139,107 |
| **29** | 4- | -C₆H₅ | B | -CH₂- | O | O | H | B | -(CH₂)₅- , | 52 - 54°C |
| **30** | 4- | -CH₃ | B | B | O | 0 | H | C(O) | -(CH₂)₄- | 84-86°C |
| **31** | 4- | -CH₃ | B | B | 0 | 0 | H | B | -(CH₂)₅- | m/z:274,121,91 |
| **32** | 4- | -C₆H₅ | B | -(CH₂)₃- | O | O | H | O | -(CH₂)₃- | 32 - 35°C |

| | | | | | | | | | | |
|---|---|---|---|---|---|---|---|---|---|---|
| B = Bindung, c = Cyclo, | | | | | | | | | | |
| Pos. A¹ = Ringposition der Gruppe A¹ relativ zu der Gruppe -Z-A²-COCF₃ | | | | | | | | | | |

### Beispiel 33:

### 6,6,6-Trifluor-1-(3-n-butoxyphenyl)hexan-1,5-dion

A) Eine Mischung aus 2,04 g 3-Hydroxybenzaldehyd, 9 g getrocknetem Kaliumcarbonat, 0,2 g Kaliumjodid und 2 ml n-Butylbromid wurden unter Schutzgasatmosphäre in 100 ml Methylethylketon 24 h lang unter Rückflußkühlung auf 80 °C erhitzt. Anschließend gab man nochmals 1 ml n-Butylbromid hinzu und erhitzte weitere 5 h lang auf 80 °C. Nach Abkühlung auf RT wurde im Wasserstrahlpumpenvakuum (= WV) eingedampft, der Rückstand mit wenig n-Hexan aufgenommen und mit 5 g Kieselgel versetzt. Das so erhaltene Gemenge wurde erneut im WV eingedampft. Der verbliebene Rückstand wurde auf eine Chromatographiesäule aufgebracht und mittels Flashchromatographie (stationäre Phase: Kieselgel, mobile Phase: Pentan/Diethylether 5:1) gereinigt. Man erhielt 2,5 g 3-n-Butoxybenzaldehyd als Öl.
B) Unter Feuchtigkeitsausschluß und Schutzgasatmosphäre wurden 500 mg des vorstehend erhaltenen Aldehyds in 5 ml trockenem THF gelöst und mit 0,05 ml einer 1M TBAF-Lösung in THF versetzt. Zu dieser Vorlage wurden langsam zunächst 0,5 ml Trimethylsilylcyanid und nach einer Stunde noch einmal 0,1 ml Trimethylsilylcyanid hinzugegeben. Dann wurde das Gemisch auf -80 °C gekühlt und es wurden bei dieser Temperatur 4 ml einer 1M Lithium-bis(trimethylsilyl)-amid-Lösung in THF hinzugegeben. Die entstandene Reaktionsmischung ließ man innerhalb von 30 Minuten auf RT auftauen und fügte anschließend 0,9 ml 4-Brom-buttersäure-ethylester sowie eine Spatelspitze Kaliumjodid hinzu. Man ließ die Reaktionsmischung 1 h lang unter Rückflußkühlung zum Sieden erhitzen, versetzte mit 10 ml einer 1M TBAF-Lösung und ließ noch 1 h lang bei RT stehen. Anschließend dampfte man das Lösungsmittel im WV ein, nahm den verbliebenen Rückstand in MTBE auf und wusch die organische Phase mit Wasser. Die organische Phase wurde abgetrennt, über Natriumsulfat getrocknet und im WV eingedampft. Der verbliebene Rückstand wurde mittels Flashchromatographie (stationäre Phase: Kieselgel; mobile Phase: n-Pentan/Ether 5:1 v/v) gereinigt. Man erhielt 320 mg 5-(3-n-Butoxyphenyl)-5-oxo-valeriansäure-ethylester, welcher ohne Charakterisierung direkt für die nachfolgende Umsetzung eingesetzt wurde.
C) 315,6 mg des vorstehend erhaltenen Ethylesters wurden in 10 ml wäßrigem Ethanol gelöst und mit 560 mg festem KOH versetzt. Die entstandene Reaktionsmischung wurde 2 h lang unter Rückflußkühlung zum Sieden erhitzt und deren Volumen wurde anschließend im WV auf etwa die Hälfte eingedampft. Nach dem Abkühlen versetzte man den verbliebenen Rückstand wurde mit 15 ml einer wäßrigen 1N Salzsäure und extrahierte die wäßrige Phase dreimal mit Diethylether. Die vereinigten Etherphasen wurden über Magnesiumsulfat getrocknet und im WV eingedampft. Nach Trocknung des verbliebenen Rückstandes im Ölpumpenvakuum (= ÖV) erhielt man 249,8 mg 5-(3-n-Butoxyphenyl)-5-oxovaleriansäure als amorphen Feststoff, welcher ohne Charakterisierung direkt für die nachfolgende Umsetzung eingesetzt wurde.
D) 240 mg der vorstehend erhaltenen Säure wurden mit 5 ml Trifluoressigsäureanhydrid auf die oben in Beispiel 4A) beschriebene Weise umgesetzt. Man erhielt 121 mg 6-(3-n-Butoxyphenyl)-3,4-dihydro-2H-pyran-2-on.
E) 1,2 g des vorstehend erhaltenen Pyranons wurden mit 0,75 ml Trifluormethyltrimethylsilan und 1,54 g TBAF-trihydrat auf die oben in Beispiel 4B) beschriebene Weise umgesetzt. Man erhielt 0,8 g der Titelverbindung als Öl, IR (Film): 2961, 1764, 1687 cm⁻¹.

### Beispiel 34:

### 6,6,6-Trifluor-1-(3-benzyloxyphenyl)hexan-1,5-dion

A) Unter Feuchtigkeitsauschluss und Schutzgasatmosphäre wurden zu 1,5 g Magnesiumspänen und 50 ml trockenem Diethylether langsam 2 ml 3-Bromanisol hinzugetropft. Nach Zugabe eines Jodkristalls wurden weitere 7,62 ml Bromanisol derart hinzugegeben, daß ein leichtes Sieden aufrechterhalten wurde. Anschließend wurde die Reaktionsmischung noch 2 h lang unter Rückflußkühlung zum Sieden erhitzt. Unter Aufrechterhalten des Siedens gab man 5,0 g Cyclopentanon hinzu und ließ noch 30 Minuten bei dieser Temperatur reagieren. Dann ließ man über Nacht bei RT stehen. Die Reaktionsmischung wurde erst mit Eis gekühlt und es wurden dann 50 g Eis und 32 ml einer kalten wäßrigen 2,5M Schwefelsäure hinzugefügt. Die wäßrige Phase wurde abgetrennt und zweimal mit je 50 ml Diethylether extrahiert. Die vereinigten organischen Phasen wurden über Natriumsulfat getrocknet und im WV eingedampft. Den verbliebenen Rückstand erhitzte man 2 h lang mit 12,5 ml einer wäßrigen 2,5 M Schwefelsäure unter Rückflußkühlung. Nach Abkühlung auf RT wurde dreimal mit je 20 ml Diethylether extrahiert und die vereinigten organischen Phasen wurden über Natriumsulfat getrocknet. Eindampfen des überschüssigen Lösungsmittels im WV und Reinigung des verbliebenen Rückstandes mittels Flashchromatographie (stationäre Phase: Kieselgel, mobile Phase: n-Heptan) lieferte 5,0 g 1-Cyclopent-1-en-1-yl-3-methoxybenzol als hellbräunlich gefärbtes Öl, welches ohne Charakterisierung direkt für die nachfolgend angegebene Umsetzung eingestzt wurde.
B) 5,2 g des vorstehend erhaltenen Cyclopentenderivates wurden mit 9,32 g vorgetrocknetem Pyridiniumchlorid 3 h lang bei 220°C gerührt. Nach Abkühlung auf RT gab man die Reaktionsmischung auf 50 ml 1N wäßrige Salzsäure. Man gab 25 ml gesättigte Kochsalzlösung hinzu und extrahierte die wäßrige Phase dreimal mit je 50 ml MTBE. Die vereinigten organischen Phasen wurden über Natriumsulfat getrocknet und im WV eingedampft. Man erhielt 4,75 g schwach hellbraunes 3-Cyclopent-1-en-1-yl-phenol welches ohne Charakterisierung direkt für die nachfolgend angegebene Umsetzung eingestzt wurde.
C) 1,0 g des vorstehend erhaltenen Cyclopentenderivates, 0,76 ml Benzylbromid, 1,34 g getrocknetes Kaliumcarbonat und 1,24 g Kaliumjodid wurden in 25 ml Aceton 5 h lang unter Rückflußkühlung zum Sieden erhitzt. Nach Abkühlung auf RT wurden 100 ml Wasser hinzugegeben und die wäßrige Phase wurde dreimal mit MTBE extrahiert. Die vereinigten organischen Phasen wurden dann der Reihe nach mit 0,5 N wäßriger Natronlauge, Wasser und gesättigter wäßriger Kochsalzlösung gewaschen, über Natriumsulfat getrocknet und im WV eingedampft. Trocknung des verbliebenen Rückstandes im ÖV lieferte 1,48 g 1-(Benzyloxy)-3-cyclopent-1-en-1-yl-benzol.
D) Unter Schutzgasatmosphäre wurde bei 0°C zu einer Lösung von 1,0 g des vorstehend erhaltenen Benzyloxycyclopentenylderivates in 5 ml Toluol tropfenweise eine Mischung von 3,5 g Kaliumpermanganat, 445 mg Aliquat® 336, 30 ml Toluol und 35 ml Wasser hinzugegeben. Nach 30-minütigem Rühren gab man zu dieser Vorlage nacheinander 1,3 g Natriumhydrogensulfit und 15 ml 6 N wäßriger Salzsäure hinzu. Man extrahierte dreimal mit je 50 ml EE, wusch die vereinigten organischen Phasen zweimal mit je 50 ml 2 N wäßriger Salzsäure und schließlich einmal mit Wasser und trocknete über Natriumsulfat. Die getrocknete organische Phase wurde im WV eingedampft und der verbliebene Rückstand wurde mittels Flashchromatographie (stationäre Phase: Kieselgel, mobile Phase: n-Heptan/Essigsäureethylester 3:1 v/v) gereinigt. Nach Eindampfen der Produktfraktionen und Trocknen im ÖV erhielt man 536 mg 5-(3-Benzyloxyphenyl)-5-oxo-valeriansäure als weißen Feststoff.
E) 1,9 g der vorstehend erhaltenen Säure wurden mit 5,4 ml Trifluoressigsäureanhydrid auf die oben in Beispiel 4A) beschriebene Weise umgesetzt. Man erhielt 1,79 g 6-(3-Benzyloxyphenyl)-3,4-dihydro-2H-pyran-2-on als braunes Öl, welches ohne weitere Reinigung oder Charakterisierung direkt für die nachfolgend angegebene Umsetzung eingesetzt wurde.
F) 1,0 g des vorstehend erhaltenen Pyranons wurden mit 0,53 ml Trifluormethyltrimethylsilan, 10 mg Cäsiumfluorid und 1,13 g TBAF-trihydrat auf die oben in Beispiel 4B) beschriebene Weise umgesetzt. Man erhielt 302 mg der Titelverbindung als Feststoff, Fp. = 75,3-80,3 °C.

### Beispiel 35:

### 6,6,6-Trifluor-1-[4-methoxy-3-(4-phenylbutyl)phenyl]hexan-1,5-dion

A) Unter Schutzgasatmosphäre wurden 4,6 g Kalium-tert.-butylat in 165 ml DMF gelöst. Unter Eiskühlung wurden zu dieser Vorlage 17,4 g (3-Phenylpropyl)-triphenylphosphoniumbromid hinzugegeben und es wurde 30 Minuten lang gerührt. Anschließend tropfte man eine Lösung von 4,44 ml 2-Methoxybenzaldehyd in 30 ml DMF hinzu und ließ die Reaktionsmischung 30 Minuten unter Eiskühlung und anschließend noch 12 h bei RT stehen. Man dampfte überschüssiges DMF im WV ein, nahm den verbliebenen Rückstand in MTBE auf und wusch die organische Phase nacheinander mit Wasser und gesättigter wäßriger Kochsalzlösung. Die organische Phase wurde über Natriumsulfat getrocknet und im WV eingedampft. Reinigung des verbliebenen Rückstandes mittels Flaschromatographie (stationäre Phase: Kieselgel, mobile Phase: n-Hexan/Essigsäureethylester 7:3 v/v) lieferte 3,5 g 1-Methoxy-2-(4-phenylbut-1-enyl)benzol.
B) 3,5 g des vorstehend erhaltenen Anisolderivates wurden in 100 ml EE gelöst, mit 0,9 g 10% Pd auf Kohle versetzt und anschließend 6 h lang unter einem Wasserstoffdruck von 4,4 bar hydriert. Nach Abfiltration des Katalysators wurde das Lösungsmittel im WV eingedampft und der verbliebene Rückstand mittels Flashchromatographie (stationäre Phase: Kieselgel, mobile Phase: n-Hexan/EE 8:2 v/v) gereinigt. Nach Eindampfen der Produktfraktionen und Trocknen im ÖV erhielt man Eindampfen der Produktfraktionen und Trocknung im ÖV lieferte 3,2 g 1-Methoxy-2-(4-phenylbutyl)benzol.
F) 3,2 g des vorstehend erhaltenen Methoxyphenylbutylbenzolderivates wurden mit 1,6 g Glutarsäureanhydrid und 3,5 g wasserfreiem Aluminiumtrichlorid auf die oben in Beispiel 2A) beschriebene Weise umgesetzt. Man erhielt 2,7 g 5-[4-Methoxy-3-(4-phenylbutyl)phenyl]-5-oxo-valeriansäure.
C) 2,7 g des vorstehend erhaltenen Valeriansäurederivates wurden mit 4,2 ml Trifluoressigsäureanhydrid auf die oben in Beispiel 4A) beschriebene Weise umgesetzt. Man erhielt 1,4 g 5-[4-Methoxy-3-(4-phenylbutyl)phenyl]-3,4-dihydro-2H-pyran-2-on.
D) 1,4 g des vorstehend erhaltenen Pyranons wurden mit 0,69 ml Trifluormethyltrimethylsilan und 1,3 g TBAF-trihydrat auf die oben in Beispiel 4B) beschriebene Weise umgesetzt. Man erhielt 1,3 g der Titelverbindung, IR (Film): 2934, 1763, 1676 cm⁻¹.

### Beispiel 37:

### 6,6,6-Trifluor-1-[4-(1-naphthyl)phenyl]hexan-1,5-dion

Unter Schutzgasatmosphäre wurden 1,0 g 1-Naphthalenboronsäure, 1,0 g Kaliumcarbonat und 0,06 g Tetrakis(triphenylphosphin)-palladium(0) in 90 ml Toluol zusammengegeben. Zu dieser Vorlage wurde eine Lösung von 1,0 g 1-(4-Bromphenyl)-6,6,6-trifluorhexan-1,5-dion in 10 ml Toluol hinzugetropft und es wurde 3 Tage lang bei 90°C gerührt. Man ließ auf RT abkühlen, tropfte 13 ml gesättigte wäßrige Natriumhydrogencarbonat-Lösung hinzu und trennte die Phasen. Die organische Phase wurde nacheinander mit Wasser, wäßriger Kaliumhydrogensulfat-Lösung, Wasser und gesättigter wäßriger Kochsalzlösung gewaschen. Man trocknete die organische Phase über Natriumsulfat und dampfte das Lösungsmittel im WV ein. Reinigung des verbliebenen Rückstandes mittels Flashchromatographie (stationäre Phase: Kieselgel, mobile Phase: n-Hexan/EE 3:2 v/v) und nachfolgende Umkristallisation der vereinigten Produktfraktionen aus EE/n-Hexan (2:3) lieferte 0,75 g der Titelverbindung, Fp. = 79,7-82,5°C.

### Beispiel 38:

### 6,6,6-Trifluor-1-(4-methoxyphenyl)-2-(4-phenoxybutyl)hexan-1,5-dion

A) 25 ml Glutarsäuremonoethylesterchlorid wurden in 250 ml trockenem Dichlormethan gelöst. Unter Eiskühlung wurden zuerst 26 ml Anisol hinzugetropft, danach gab man portionsweise 42,4 g Aluminiumtrichlorid derart zu, daß die Reaktionstemperatur 10°C nicht überstieg. Die rot gefärbte Lösung wurde 3 h lang bei 0°C gerührt und dann auf mit Eis versetzte, verdünnte wäßrige Salzsäure gegossen. Die organische Phase wurde abgetrennt, der Reihe nach mit Wasser und mit gesättigter wäßriger Kochsalzlösung gewaschen und über Natriumsulfat getrocknet. Man dampfte die organische Phase im WV ein und kristallisierte den verbliebenen Rückstand aus MTBE um. Man erhielt 38,8 g 5-(4-Methoxyphenyl)-5-oxo-valeriansäureethylester, Fp. = 56,6-58,1°C.
B) 4,0 g des vorstehend erhaltenen Valeriansäureesters wurden unter Schutzgasatmosphäre in 100 ml trockenem DMF gelöst. Dazu gab man unter Eiskühlung 0,7 g Natriumhydrid (60%ig) und ließ noch 30 Minuten bei 0°C rühren. Zu dieser Vorlage tropfte man eine Lösung von 4,2 g 4-Phenoxy-1-brombutan in 40 ml trockenem DMF bei 0-5°C hinzu. Anschließend wurde die Reaktionsmischung 1 h lang bei 0°C, 1 h lang bei RT und schließlich 2 h lang bei 50°C gerührt. Nach Abkühlung auf RT wurde weitere 16 h lang stehengelassen, bevor man das Reaktionsgemisch auf mit Eis vermischte gesättigte wäßrige Kaliumhydrogensulfat-Lösung goß und die wäßrige Phase zweimal mit je 70 ml EE extahierte. Die vereinigten organischen Phasen wurden der Reihe nach mit Wasser und mit gesättigter wäßriger Kochsalzlösung gewaschen, über Natriumsulfat getrocknet und im WV eingedampft. Der verbliebene Rückstand wurde mittels Flashchromatographie (stationäre Phase: Kieselgel, mobile Phase: n-Hexan, das allmählich durch EE ersetzt wurde) gereinigt. Die vereinigten Produktfraktionen wurden im WV eingedampft und am Kugelrohr bei 250 °C und 2 mbar destilliert. Man erhielt 3,0 g Ethyl-4-(4-methoxyphenyl)-8-phenoxyoctanoat.
C) 3,0 g des vorstehend erhaltenen Ethylmethoxyphenylphenoxyoctanoats wurden in 20 ml Methanol gelöst und mit einer Lösung von 8,5 g festem KOH in.30 ml Wasser versetzt. Man ließ die Reaktionsmischung 16 h lang bei RT rühren und extrahierte diese anschließend mit 100 ml MTBE. Die wäßrige Phase wurde mit verdünnter wäßriger Salzsäure angesäuert und dreimal mit je 70 ml MTBE extrahiert. Anschließend wusch man die organische Phase der Reihe nach mit Wasser und mit gesättigter wäßriger Kochsalzlösung, trocknete über Natriumsulfat und dampfte im WV ein. Der verbliebene Rückstand wurde mittels Flashchromatographie (stationäre Phase: Kieselgel, mobile Phase: zuerst n-Hexan/EE 4:1, welches allmählich durch reinen EE ersetzt wurde) gereinigt. Trocknung der Produktfraktionen lieferte 1,8 g 4-(4-Methoxybenzoyl)-8-phenoxyoctansäure.
D) 1,6 g des vorstehend erhaltenen Methoxybenzoylphenoxyoctansäure-Derivates wurden mit 0,66 ml Trifluoressigsäureanhydrid auf die oben in Beispiel 4A) beschriebene Weise umgesetzt. Man erhielt 1,2 g 6-(4-Methoxyphenyl)-5-(4-phenoxybutyl)-3,4-dihydro-2H-pyran-2-on als Öl, IR (Film): 2937, 1764, 1586 cm⁻¹.
E) 1,5 g des vorstehend erhaltenen Pyranons wurden mit 0,66 ml Trifluormethyltrimethylsilan und 1,34 g TBAF-trihydrat auf die oben in Beispiel 4B) beschriebene Weise umgesetzt. Man erhielt 1,5 g der Titelverbindung, IR (Film): 2941, 1763, 1670 cm⁻¹.
Nach den vorstehend angegebenen Verfahren oder analog zu diesen Verfahren können auch die in der nachstehenden Tabelle 2 angegebenen Verbindungen der Formel I hergestellt werden.

**Tabelle 2:**

| Weitere Verbindungen der Formel I: | | | | | | | | | | |
|---|---|---|---|---|---|---|---|---|---|---|
| **Bsp** | **A**^{**1**} | | | | | | **R**^{**2**} | **Z** | **A**^{**2**} | **Fp.[°C]; Ms m/z; IR[cm**^{**-1**}**]** |
| | **Pos A1** | **R**¹ | **W** | **A**^{**3**} | **y** | **n** | | | | |
| **39** | 4- | -C₆H₅ | B | -(CH₂)₃- | O | 0 | H | O | -(CH₂)₅- | 46-50 °C |
| **40** | 4- | -CH₃ | B | -(CH₂)₇- | 0 | 0 | H | 0 | -(CH₂)₃- | 37-40°C |
| **41** | 4- | c-C₆H₁₁ | B | -CH₂- | O | 0 | H | 0 | -(CH₂)₃- | 2926, 1764, 1509 cm⁻¹ |
| **42** | 4- | -C₆H₅ | O | -(CH₂)₄- | O | 0 | H | O | -(CH₂)₃- | 104-107 °C |
| **43** | 4- | -C₆H₅ | B | -CH₂- | O | 0 | H | C(O) | -(CH₂)₃- | 72,3-73,1 °C |
| **44** | 4- | -CH₃ | B | B | O | O | H | C(O) | (n-C₅H₁₁-)CH-(CH₂)₂- | 2933,1764,1671 cm⁻¹ |
| **45** | 4- | -C₆H₅ | B | -(CH₂)₃- | O | 0 | H | B | -(CH₂)₄- | 2938, 1764, 1512 cm⁻¹ |
| **46** | 3- | -CH₃ | B | -(CH₂)₁₀- | O | 0 | H | B | -(CH₂)₄- | 2855, 1765, 1608 cm⁻¹ |
| **47** | 4- | -CH₃ | B | B | O | 0 | H | C(O) | (C₆H₅-n-C₃H₆-)CH-(CH₂)₂- | 2936, 1763, 1670 cm⁻¹ |
| **48** | 4- | -CH(CH₃)₂ | B | -(CH₂)₃- | B | 0 | H | B | -(CH₂)₄- | 2930,1765,1514 cm⁻¹ |
| **49** | 4- | -C₆H₅ | O | -(CH₂)₄- | O | 0 | H | B | -(CH₂)₄- | 21-30 °C |
| **50** | 3- | -CH(CH₃)₂ | B | -(CH₂)₃- | B | 0 | H | B | -(CH₂)₄- | 2932, 1765, 1607 cm⁻¹ |
| **51** | 4- | -CH₃ | B | B | O | 0 | H | C(O) | (n-C₁₀H₂₁-)CH-(CH₂)₂- | 2927,1764,1670 cm⁻¹ |
| **52** | 4- | -CH₃ | B | -(CH₂)₁₀- | B | 0 | H | B | -(CH₂)₄- | 2926,1765,1514 cm⁻¹ |
| **53** | 4- | -C₆H₅ | B | -(CH₂)₄- | B | 0 | H | B | -(CH₂)₄- | 2932,1763,1604 cm⁻¹ |
| **54** | 4- | -C₆H₅ | O | -(CH₂)₄- | O | 0 | H | C(O) | *-*(CH₂)₃*-* | 78,8-60,4 °C |
| **55** | 4- | -C₆H₅ | B | -(CH₂)₃- | O | 0 | H | C(O) | -(CH₂)₃- | 52,6-54,5 °C |
| **56** | 4- | -C₆H₅ | B | B | B | O | H | B | -(CH₂)₄- | 2935,1763,1709 cm⁻¹ |
| **57** | | H | B | B | B | 0 | 4-Br | C(O) | -(CH₂)₃- | 1764,1687,1586 cm⁻¹ |
| **58** | 3- | -C₆H₅ | B | -(CH₂)₂- | B | 0 | 6-OCH₃ | C(O) | -(CH₂)₃- | 2944,1763,1671 cm⁻¹ |
| **59** | 3- | -CH(CH₃)₂ 2 | B | -(CH₂)₃- | B | O | 6-OCH₃ 3 | C(O) | -(CH₂)₃- | 35,3-36,5°C |
| **60** | 4- | (4)-(CH₂)₄- (3) | | | | | | C(O) | -(CH₂)₃- | 2936,1763,1681,1605, 1572 cm⁻¹ |
| **61** | 4- | (4)-O-(CH₂)₂- (3) | | | | | | C(O) | -(CH₂)₃- | 68,8 - 69,5 °C |
| **62** | 4- | 2-Naphthyl | B | B | B | O | H | C(O) | -(CH₂)₃- | 1761,1673,1606 cm⁻¹ |
| **63** | 4- | -CH₃ | B | -(CH₂)₁₅- | O | O | H | C(O) | -(CH₂)₃- | 67,7 - 68,9 °C |
| **64** | 3- | -CH₃ | B | -(CH₂)₁₀- | B | O | 4-OCH₃ | C(O) | -(CH₂)₃- | 2925,1764,1674 cm⁻¹ |
| **65** | 4- | -CH₃ | B | -(CH₂)₁₃- | 0 | 0 | H | C(O) | -(CH₂)₃- | 63,3 - 65,0 °C |

| | | | | | | | | | | |
|---|---|---|---|---|---|---|---|---|---|---|
| B = Bindung, c = Cyclo, | | | | | | | | | | |
| Pos. A¹ = Ringposition der Gruppe A¹ relativ zu der Gruppe -Z-A²-COCF₃ | | | | | | | | | | |

### Beispiel I:

### 6,6,6-Trifluor-1-(4-methoxyphenyl)hexan-1,5-dion enthaltende Kapseln

Es werden Kapseln in folgender Zusammensetzung pro Kapsel hergestellt:

| | |
|---|---|
| 6,6,6-Trifluor-1-(4-methoxyphenyl)hexan-1,5-dion | 20 mg |
| Maisstärke | 60 mg |
| Milchzucker | 300 mg |
| Ethylacetat | q.s. |

Der Wirkstoff, die Maisstärke und der Milchzucker werden unter Zuhilfenahme von Ethylacetat zu einer homogenen pastösen Mischung verarbeitet. Die Paste wird zerkleinert und das entstehende Granulat wird auf ein geeignetes Blech gebracht und zur Entfernung des Lösungsmittels bei 45 °C getrocknet. Das getrocknete Granulat wird durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann in 400 mg fassenden Kapseln (= Kapselgröße 0) abgefüllt.

## Patentansprüche

1. Verwendung von Verbindungen der allgemeinen Formel I, 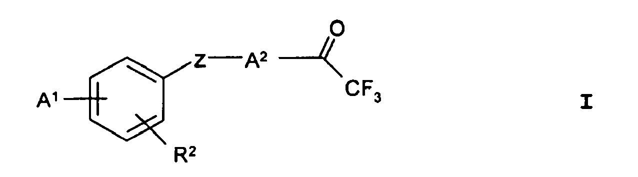 worin
A¹ eine Gruppe der Formel R¹-W-A³-Y-(CH₂)ₙ- bedeutet, worin
R¹ Wasserstoff,
C₁₋₄-Alkyl,
C₃₋₇-Cycloalkyl,
Phenyl-C₀₋₄-alkyl, welches im Phenylring gegebenenfalls durch C₁₋₄-Alkylendioxy oder ein- bis zweifach durch C₁₋₉-Alkyl, C₁₋₉-Alkoxy, Halogen oder Perfluor-C₁₋₄-alkyl substituiert ist, oder Naphthyl bedeutet,
W eine Bindung oder Sauerstoff bedeutet,
A³ eine Bindung oder C₁₋₂₀-Alkylen bedeutet, welches gegebenenfalls ein- bis zweifach durch Phenyl, Naphthyl, C₁₋₄-Alkyl oder C₅₋₇-Cycloalkyl substituiert ist,
Y eine Bindung oder Sauerstoff bedeutet und
n eine ganze Zahl von 0 bis 3 bedeutet, und
R² Wasserstoff, C₁₋₄-Alkyl, C₁₋₄-Alkoxy oder Halogen bedeutet, oder
A¹ und R² gemeinsam mit den Kohlenstoffatomen, an welche sie gebunden sind, eine C₅₋₇-Cycloalkylgruppe bilden, deren sp³-hybridisierte Kohlenstoffatome gegebenenfalls ein- bis zweifach durch Sauerstoff ersetzt sind,
Z eine Bindung, Sauerstoff oder Carbonyl bedeutet und
A² C₁₋₂₀-Alkylen bedeutet, welches gegebenenfalls einfach durch C₁₋₁₂-Alkyl, C₁₋₁₂-Alkyl-phenyl oder C₁₋₁₂-Alkyl-oxyphenyl substituiert ist, bedeutet,
sowie von Solvaten und/oder Hydraten der Verbindungen der Formel I und von Gemischen dieser Verbindungen, zur Herstellung pharmazeutischer Zubereitungen für die Behandlung und/oder Prophylaxe von Obesitas sowie dem Metabolischen Syndrom, welches insbesondere Bluthochdruck, Insulinresistenz, Dyslipoproteinämie und Hyperurikämie umfaßt.

2. Verwendung von Verbindungen der allgemeinen Formel If 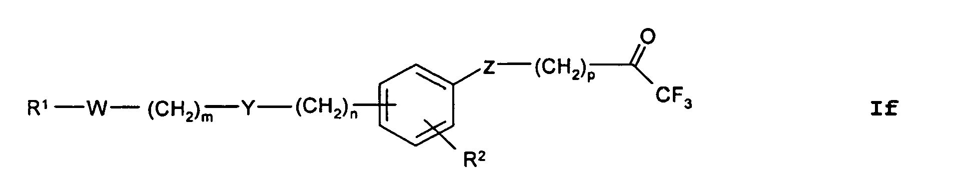 nach Anspruch 1,
worin
R¹ Wasserstoff,
C₁₋₄-Alkyl,
C₃₋₇-Cycloalkyl,
Phenyl-C₀₋₄-alkyl, welches im Phenylring gegebenenfalls durch C₁₋₄-Alkylendioxy oder ein- bis zweifach durch C₁₋₄-Alkyl, C₁₋₄-Alkoxy, Halogen oder Perfluor-C₁₋₄₋alkyl substituiert ist, oder
Naphthyl bedeutet,
R² Wasserstoff, C₁₋₄-Alkyl, C₁₋₄-Alkoxy oder Halogen bedeutet,
W eine Bindung oder Sauerstoff bedeutet,
Y eine Bindung oder Sauerstoff bedeutet,
Z eine Bindung, Sauerstoff oder Carbonyl bedeutet,
m eine ganze Zahl von 0 bis 10 bedeutet
n eine ganze Zahl von 0 bis 3 bedeutet und
p eine ganze Zahl von 1 bis 20 bedeutet.

3. Verwendung nach Anspruch 1 von Verbindungen der allgemeinen Formel I, worin R² Wasserstoff oder Halogen bedeutet.

4. Verwendung nach Anspruch 1 von Verbindungen der allgemeinen Formel I, worin die Gruppe A¹ in para-Stellung zu dem Rest "-Z-A²-C(O)-CF₃" angeordnet ist.

5. Verwendung nach Anspruch 1 von Verbindungen, welche ausgewählt sind aus der Gruppe bestehend aus 1,1,1-Trifluor-9-phenyl-nonan-2-on und 1,1,1-Trifluor-8-phenyl-octan-2-on.

6. Verbindungen, welche ausgewählt sind aus der Gruppe bestehend aus 5-[4-(Benzyloxymethyl)-phenoxy]-1,1,1-trifluorpentan-2-on; 5-[4-(Benzyloxy)phenoxy]-1,1,1-trifluorpentan-2-on; 1,1,1-Trifluor-12-phenoxy-dodecan-2-on; 1,1,1-Trifluor-5-[4-(3-phenylpropoxy)phenoxy]pentan-2-on; 6-(4-Methoxyphenyl)-1,1,1-trifluorhexan-2-on und 1,1,1-Trifluor-11-phenyl-undecan-2-on.

7. Verbindungen der allgemeinen Formel Ig, 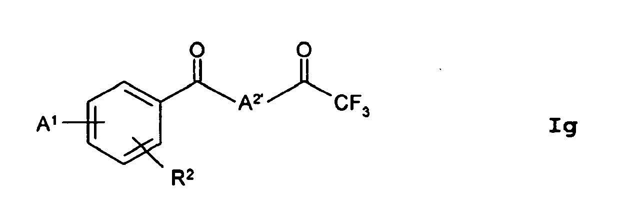 worin
A¹ eine Gruppe der Formel R¹-W-A³-Y-(CH₂)ₙ- bedeutet, worin
R¹ Wasserstoff,
C₁₋₄-Alkyl,
C₃₋₇-Cycloalkyl,
Phenyl-C₀₋₄-alkyl, welches im Phenylring gegebenenfalls durch C₁₋₄-Alkylendioxy oder ein- bis zweifach durch C₁₋₄-Alkyl, C₁₋₄-Alkoxy, Halogen oder Perfluor-C₁₋₄-alkyl substituiert ist, oder Naphthyl bedeutet,
W eine Bindung oder Sauerstoff bedeutet,
A³ eine Bindung oder C₁₋₂₀-Alkylen bedeutet, welches gegebenenfalls ein- bis zweifach durch Phenyl, Naphthyl, C₁₋₄-Alkyl oder C₅₋₇-Cycloalkyl substituiert ist,
Y eine Bindung oder Sauerstoff bedeutet und
n eine ganze Zahl von 0 bis 3 bedeutet, und
R² Wasserstoff, C₁₋₄-Alkyl, C₁₋₄-Alkoxy oder Halogen bedeutet, oder
A¹ und R² gemeinsam mit den Kohlenstoffatomen, an welche sie gebunden sind, eine C₅₋₇-Cycloalkylgruppe bilden, deren sp³-hybridisierte Kohlenstoffatome gegebenenfalls ein- bis zweifach durch Sauerstoff ersetzt sind, und
A^{2'} n-Propylen, welches gegebenenfalls einfach durch C₁₋₁₂₋Alkyl, C₁₋₁₂-Alkyl-phenyl oder C₁₋₁₂-Alkyl-oxyphenyl substituiert ist, bedeutet,
sowie Solvate und Hydrate von Verbindungen der Formel Ig zur Verwendung als Arzneimittel.

8. Verbindungen nach Anspruch 7, welche ausgewählt sind aus der Gruppe bestehend aus 6,6,6-Trifluor-1-(4-methoxyphenyl)hexan-1,5-dion; 6,6,6-Trifluor-1-(4-(4-phenoxybutoxy)-phenyl)hexan-1,5-dion; 6,6,6-Trifluor-1-(4-(3-phenylpropoxy)-phenyl)hexan-1,5-dion; 1-(4-Bromphenyl)-6,6,6-trifluorhexan-1,5-dion; 6,6,6-Trifluor-1-(4-(1-naphthyl)phenyl)hexan-1,5-dion; 6,6,6-Trifluor-1-(5,6,7,8-tetrahydronaphthalen-2-yl)hexan-1,5-dion; 6,6,6-Trifluor-1-(4-(4-methoxy-1-naphthyl)phenyl)hexan-1,5-dion; 6,6,6-Trifluor-1-(4-(2-naphthyl)phenyl)hexan-1,5-dion; 6,6,6-Trifluor-1-(4-(hexadecyloxy)phenyl)hexan-1,5-dion und 6,6,6-Trifluor-1-(4-(tetradecyloxy)phenyl)hexan-1,5-dion.

9. Verbindungen der allgemeinen Formel Id, 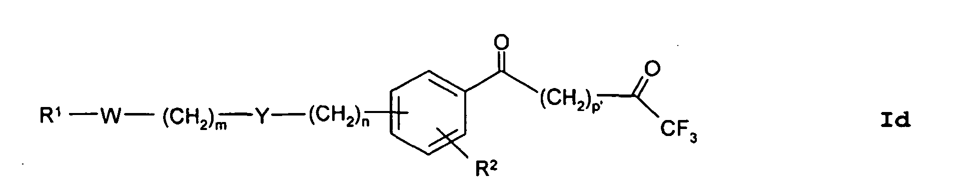 zur Verwendung als Arzneimittel nach Anspruch 7,
worin
R¹ Wasserstoff,
C₁₋₄-Alkyl,
C₃₋₇-Cycloalkyl,
Phenyl-C₀₋₄-alkyl, welches im Phenylring gegebenenfalls durch C₁₋₄-Alkylendioxy oder ein- bis zweifach durch C₁₋₄-Alkyl, C₁₋₄-Alkoxy, Halogen oder Perfluor-C₁₋₄₋alkyl substituiert ist, oder
Naphthyl bedeutet,
R² Wasserstoff, C₁₋₄-Alkyl, C₁₋₄-Alkoxy oder Halogen bedeutet,
W eine Bindung oder Sauerstoff bedeutet,
Y eine Bindung oder Sauerstoff bedeutet,
m eine ganze Zahl von 0 bis 10 bedeutet
n eine ganze Zahl von 0 bis 3 bedeutet und
p' 3 bedeutet.

10. Verbindungen zur Verwendung als Arzneimittel, welche ausgewählt sind aus der Gruppe bestehend aus 1,1,1-Trifluor-7-phenyl-heptan-2-on und 5-(4-Methoxyphenyl)-1,1,1-trifluorpentan-2-on.

11. Verfahren zur Herstellung von Verbindungen der allgemeinen Formel I", 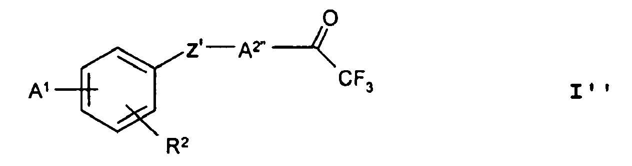 worin
A¹ eine Gruppe der Formel R¹-W-A³-Y-(CH₂)ₙ- bedeutet, worin
R¹ Wasserstoff,
C₁₋₄-Alkyl,
C₃₋₇-Cycloalkyl,
Phenyl-C₀₋₄-alkyl, welches im Phenylring gegebenenfalls durch C₁₋₄-Alkylendioxy oder ein- bis zweifach durch C₁₋₄-Alkyl, C₁₋₄-Alkoxy, Halogen oder Perfluor-C₁₋₄-alkyl substituiert ist, oder Naphthyl bedeutet,
W eine Bindung oder Sauerstoff bedeutet,
A³ eine Bindung oder C₁₋₂₀-Alkylen bedeutet, welches gegebenenfalls ein- bis zweifach durch Phenyl, Naphthyl, C₁₋₄-Alkyl oder C₅₋₇-Cycloalkyl substituiert ist,
Y eine Bindung oder Sauerstoff bedeutet und
n eine ganze Zahl von 0 bis 3 bedeutet, und
R² Wasserstoff, C₁₋₄-Alkyl, C₁₋₄-Alkoxy oder Halogen bedeutet, oder
A¹ und R² gemeinsam mit den Kohlenstoffatomen, an welche sie gebunden sind, eine C₅₋₇-Cycloalkylgruppe bilden, deren sp³-hybridisierte Kohlenstoffatome gegebenenfalls ein- bis zweifach durch Sauerstoff ersetzt sind,
Z' Carbonyl bedeutet und
A^{2"} C₁₋₃-Alkylen bedeutet, welches gegebenenfalls einfach durch C₁₋₁₂-Alkyl, C₁₋₁₂-Alkyl-phenyl oder C₁₋₁₂-Alkyl-oxyphenyl substituiert ist,
**dadurch gekennzeichnet, daß** man eine Verbindung der allgemeinen Formel XIb" 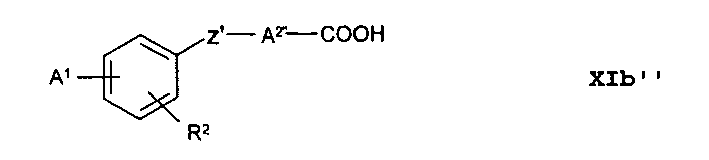 worin A¹, A^{2"}, R² und Z' obige Bedeutungen besitzen, mit Trifluoressigsäureanhydrid oder Essigsäureanhydrid umsetzt und als Zwischenprodukte erhaltene cyclische En-Lactone mit Trifluormethyltrimethylsilan umsetzt.

## Claims

1. The use of compounds of the general formula I, 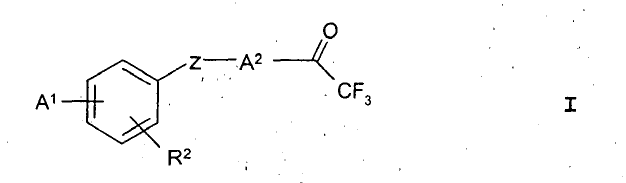 wherein
A¹ is a group of the formula R¹-W-A³-Y-(CH₂)ₙ-, wherein
R¹ is hydrogen,
C₁₋₄-alkyl,
C₃₋₇-cycloalkyl,
phenyl-C₀₋₄-alkyl which is optionally substituted in the phenyl ring by C₁₋₄-alkylenedioxy or one to two times by C₁₋₄-alkyl,C₁₋₄-alkoxy, halogen or perfluoro-C₁₋₄-alkyl, or
naphthyl,
W is a bond or oxygen,
A³ is a bond or C₁₋₂₀-alkylene which is optionally substituted one to two times by phenyl, naphthyl, C₁₋₄-alkyl or C₅₋₇-cycloalkyl,
Y is a bond or oxygen and
n is a whole number from 0 to 3, and
R² is hydrogen, C₁₋₄-alkyl, C₁₋₄-alkoxy or halogen, or
A¹ and R², together with the carbon atoms to which they are bonded, form a C₅₋₇-cycloalkyl group, the sp³-hybridised carbon atoms of which,are optionally replaced one to two times by oxygen,
Z is a bond, oxygen or carbonyl and
A² is C₁₋₂₀-alkylene which is optionally substituted once by C₁₋₁₂-alkyl, C₁₋₁₂-alkyl-phenyl or C₁₋₁₂₋alkyloxyphenyl,
and of solvates and/or hydrates of the compounds of Formula I and of mixtures of these compounds for the preparation of pharmaceutical preparations for the treatment and/or prophylaxis of obesity and metabolic syndrome, which covers in particular hypertension, insulin resistance, dyslipoproteinaemia and hyperuricaemia.

2. The use of compounds of the general formula If 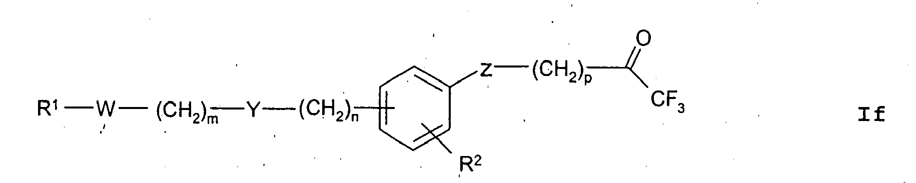 according to Claim 1,
wherein
R¹ is hydrogen,
C₁₋₄-alkyl,
C₃₋₇-cycloalkyl,
phenyl-C₀₋₄-alkyl which is optionally substituted in the phenyl ring by C₁₋₄-alkylenedioxy or one to two times by C₁₋₄-alkyl, C₁₋₄-alkoxy, halogen or perfluoro-C₁₋₄-alkyk, or
naphthyl,
R² is hydrogen, C₁₋₄-alkyl, C₁₋₄-alkoxy or halogen,
W is a bond or oxygen,
Y is a bond or oxygen,
Z is a bond, oxygen or carbonyl,
m is a whole number from 0 to 10,
n is a whole number from 0 to 3, and
p is a whole number from 1 to 20.

3. The use according to Claim 1 of compounds of the general formula I, wherein R² is hydrogen or halogen.

4. The use according to Claim 1 of compounds of the general formula I, wherein the group A¹ is located in the para position relative to the radical "-Z-A²-C(O)-CF₃".

5. The use according to Claim 1 of compounds which are selected from the group consisting of 1,1,1-trifluoro-9-phenyl-nonan-2-one and 1,1,1-trifluoro-8-phenyl-octan-2-one.

6. Compounds which are selected from the group consisting of 5-[4-(benzyloxymethyl)-phenoxy]-1,1,1-trifluoropentan-2-one; 5-[4-(benzyloxy)phenoxy]-1,1,1-trifluoropentan-2-one; 1,1,1-trifluoro-12-phenoxy-dodecan-2-one; 1,1,1-trifluoro-5-[4-(3-phenylpropoxy)phenoxy]pentan-2-one; 6-(4-methoxyphenyl)-1,1,1-trifluorohexan-2-one and 1,1,1-trifluoro-11-phenyl-undecan-2-one.

7. Compounds of the general formula Ig, 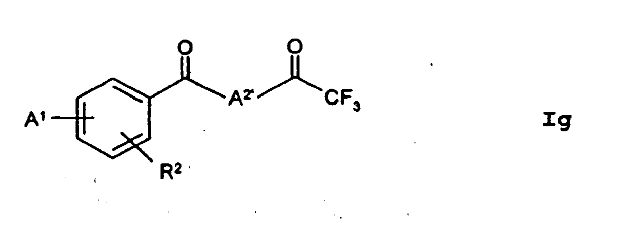 wherein
A¹ is a group of the formula R¹-W-A³-Y-(CH₂)ₙ-, wherein
R¹ is hydrogen,
C₁₋₄-alkyl,
C₃₋₇-cycloalkyl,
phenyl-C₀₋₄-alkyl which is optionally substituted in the phenyl ring by C₁₋₄-alkylenedioxy or one to two times by C₁₋₄-alkyl, C₁₋₄-alkoxy, halogen or perfluoro-C₁₋₄-alkyl, or
naphthyl,
W is a bond or oxygen,
A³ is a bond or C₁₋₂₀-alkylene which is optionally substituted one to two times by phenyl, naphthyl, C₁₋₄-alkyl or C₅₋₇-cycloalkyl,
Y is a bond or oxygen and
n is a whole number from 0 to 3, and
R² is hydrogen, C₁₋₄-alkyl, C₁₋₄-alkoxy or halogen, or
A¹ and R², together with the carbon atoms to which they are bonded, form a C₅₋₇-cycloalkyl group, the sp³-hybridised carbon atoms of which are optionally replaced one to two times by oxygen, and
A^{2'} is n-propylene which is optionally substituted once by C₁₋₁₂-alkyl, C₁₋₁₂-alkyl-phenyl or C₁₋₁₂-alkyl-oxyphenyl,
and solvates and hydrates of compounds of Formula Ig for use as medicaments.

8. Compounds according to Claim 7, which are selected from the group consisting of 6,6,6-trifluoro-1-(4-methoxyphenyl)hexane-1,5-dione; 6,6,6-trifluoro-1-(4-(4-phenoxybutoxy)phenyl)hexane-1,5-dione; 6,6,6-trifluoro-1-(4-(3-phenylpropoxy)phenyl)hexane-1,5-dione; 1-(4-bromophenyl)-6,6,6-trifluorohexane-1,5-dione; 6,6,6-trifluoro-1-,(4-(1-naphthyl)phenyl)hexane-1,5-dione; 6,6,6-trifluoro-1-(5,6,7,8-tetrahydronaphthalen-2-yl)heXane-1,5-dione; 6,6,6-trifluoro-1-(4-(4-methoxy-1-naphthyl)phenyl)hexane-1,5-dione; 6,6,6-trifluoro-1-(4-(2-naphthyl)phenyl)hexane-1,5-dione; 6,6,6-trifluoro-1-(4-(hexadecyloxy)phenyl)hexane-1,5-dione and 6,6,5-trifluoro-1-(4-(tetradecyloxy)phenyl)hexane-1,5-dione.

9. Compounds of the general formula Id, 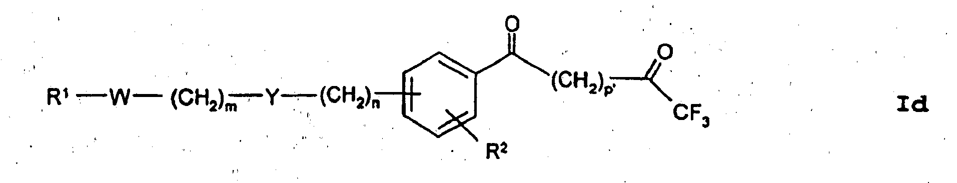 for use as medicaments according to Claim 7,
wherein
R¹ is hydrogen,
C₁₋₄-alkyl,
C₃₋₇-cycloalkyl,
phenyl-C₀₋₄-alkyl which is optionally substituted in the phenyl ring by C₁₋₄-alkylenedioxy or one to two times by C₁₋₄-alkyl, C₁₋₄-alkoxy, halogen or perfluoro-C₁₋₄-alkyl, or naphthyl,
R² is hydrogen, C₁₋₄-alkyl, C₁₋₄-alkoxy or halogen,
W is a bond or oxygen,
Y is a bond or oxygen,
m is a whole number from 0 to 10,
n is a whole number from 0 to 3, and
p' is 3.

10. Compounds for use as medicaments, which are selected from the group consisting of 1,1,1-trifluoro-7-phenyl-heptan 2-one and 5-(4-methoxyphenyl)-1,1,1-trifluoropentan-2-one.

11. A process for the preparation of compounds of the general formula I'', 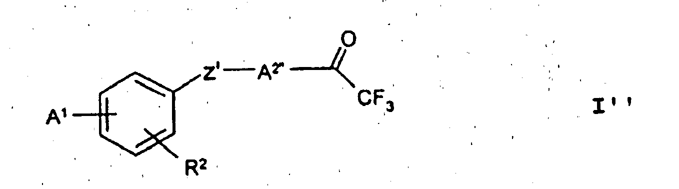 wherein
A¹ is a group of the formula R¹-W-A³-Y-(CH₂)ₙ-, wherein
R¹ is hydrogen,
C₁₋₄-alkyl,
C₃₋₇-cycloalkyl,
phenyl-C₀₋₄-alkyl which is optionally substituted in the phenyl ring by C₁₋₄-alkylenedioxy or one to two times by C₁₋₄-alkyl, C₁₋₄-alkoxy, halogen or perfluoro-C₁₋₄-alkyl, or
naphthyl,
W is a bond or oxygen,
A³ is a bond or C₁₋₂₀-alkylene which is optionally substituted one to two times by phenyl, naphthyl, C₁₋₄-alkyl or C₅₋₇-cycloalkyl,
Y is a bond or oxygen and
n is a whole number from 0 to 3, and
R² is hydrogen, C₁₋₄-alkyl, C₁₋₄-alkoxy or halogen, or
A¹ and R², together with the carbon atoms to which they are bonded, form a C₅₋₇-cycloalkyl group, the sp³-hybridised carbon atoms of which are optionally replaced one to two times by oxygen,
Z' is carbonyl and
A^{2"} is C₁₋₃-alkylene which is optionally substituted once by C₁₋₁₂-alkyl, C₁₋₁₂-alkyl-phenyl or C₁₋₁₂-alkyl-oxyphenyl,
**characterised in that**
a compound of the general formula XIb'' 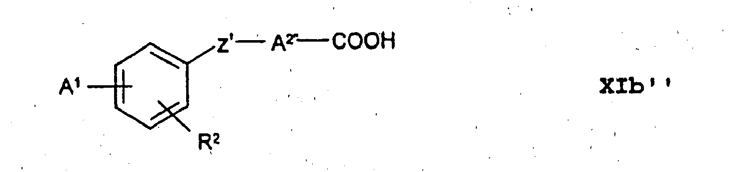 wherein A¹, A^{2"}, R² and Z' have the above meanings, is reacted with trifluoroacetic anhydride or acetic anhydride and cyclic En-lactones obtained as intermediate products are reacted with (trifluoromethyl) trimethylsilane.

## Revendications

1. Utilisation de composés de formule générale I, 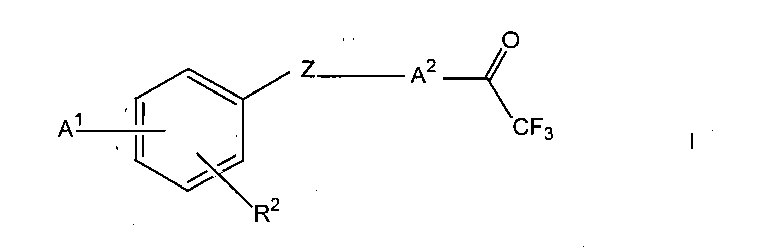 dans laquelle
A¹ représente un groupe de formule R¹-W-A³-Y-(CH₂)ₙ, où
R¹ représente un atome d'hydrogène, un alkyle en C₁ à C₄ un cycloalkyle en C₃ à C₇, un alkyl en C₀ à C₄-phényle, éventuellement substitué dans le cycle phényle par un alkylènedioxy en C₁ à C₄, ou mono- ou bi-substitué par un alkyle en C₁ à C₄, un alcoxy en C₁ à C₄, un halogène ou un perfluoroalkyle en C₁ à C₄, ou encore représente un naphtyle,
W représente une liaison ou un atome d'oxygène,
A³ représente une liaison ou un alkylène en C₁ à C₂₀, éventuellement mono- ou bi-substitué par un phényle, un naphtyle, un alkyle en C₁ à C₄, ou un cycloalkyle en C₅ à C₇,
Y représente une liaison ou un atome d'oxygène, et
n représente un nombre entier de 0 à 3, et
R² représente un atome d'hydrogène, un alkyle en C₁ à C₄, un alcoxy en C₁ à C₄, ou un halogène, ou
A¹ et R², conjointement avec les atomes de carbone auxquels ils sont liés, représentent un groupe cycloalkyle en C₅ à C₇, dont les atomes de carbone hybridés sp3 sont éventuellement remplacés une ou deux fois par un atome d'oxygène,
Z représente une liaison, un atome d'oxygène ou un groupe carbonyle et
A² représente un alkylène en C₁ à C₂₀, qui est éventuellement monosubstitué par un alkyle en C₁ à C₁₂, un alkyl en C₁ à C₁₂-phényle ou un alkyl en C₁ à C₁₂-oxyphényle,
ainsi que de solvates et/ou d'hydrates des composés de formule I et de mélanges de ces composés, pour l'obtention de préparations pharmaceutiques pour le traitement et/ou la prophylaxie de l'obésité ainsi que d'un syndrome métabolique comprenant en particulier l'hypertension artérielle, l'insulinorésistance, la dyslipoprotéinémie et l'hyperuricémie.

2. Utilisation de composés de formule générale If, 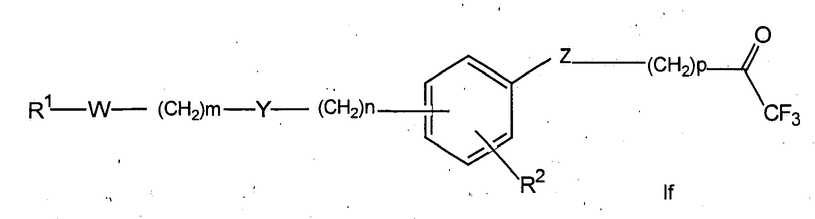 Selon la revendication 1 dans laquelle
R¹ représente un atome d'hydrogène, un alkyle en C₁ à C₄, un cycloalkyle en C₃ à C₇, un alkyl en C₀ à C₄-phényle, éventuellement substitué dans le cycle phényle par un alkylènedioxy en C₁ à C₄, ou mono- ou bi-substitué par un alkyle en C₁ à C₄, un alcoxy en C₁ à C₄, un halogène ou un perfluroralkyle en C₁ à C₄, ou encore représente un naphtyle,
R² représente un atome d'hydrogène, un alkyle en C₁ à C₄, un alcoxy en C₁ à C₄, ou un halogène,
W représente une liaison ou un atome d'oxygène,
Y représente une liaison ou un atome d'oxygène,
Z représente une liaison, un atome d'oxygène ou un groupe carbonyle,
m est un nombre entier de 0 à 10,
n est un nombre entier de 0 à 3, et
p est un nombre entier de 1 à 20.

3. Utilisation selon la revendication 1 de composés de formule générale I, dans laquelle R² représente un atome d'hydrogène ou un halogène.

4. Utilisation selon la revendication 1 de composés de formule générale I, dans laquelle le groupe A¹ est situé en position para par rapport au groupe de formule "-Z-A² -C(O)-CF₃".

5. Utilisation selon la revendication 1 de composés choisis dans le groupe constitué de la 1,1,1-trifluor-9-phényl-nonan-2-one et de la 1,1,1-trifluoro-8-phényl-octan-2-one.

6. Composés choisis dans le groupe constitué de la 5-[4-(benzyloxyméthyl)-phénoxy]-1,1,1-trifluoropentan-2-one ; de la 5-(4-(benzyloxy)phénoxy]-1,1,1-trifluoropentan-2-one ; de la 1,1,1-trifluor-12-phénoxy-dodécan-2-one ; de la 1,1,1-trifluoro-5-[4-(3-phénylpropoxy)phénoxy]]pentan-2-one ; de la 6-(4-méthoxyphényl)-1,1,1-trifluorohexan-2-one et de la 1,1,1-trifluoro-11-phényl-undécan-2-one.

7. Composés de formule générale Ig, 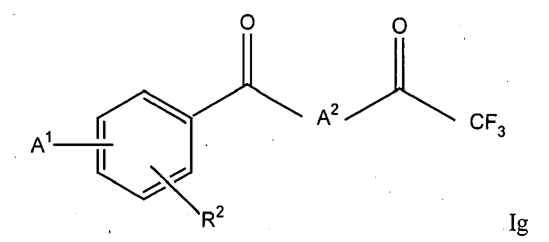 dans laquelle
A¹ représente un groupe de formule R¹-W-A³-Y-(CH₂)ₙ, où
R¹ représente un atome d'hydrogène, un alkyle en C₁ à C₄, un cycloalkyle en C₃ à C₇, un alkyl en C₀ à C₄-phényle, éventuellement substitué dans le cycle phényle par un alkylènedioxy en C₁ à C₄, ou mono- ou bi-substitué par un alkyle en C₁ à C₄, un alcoxy en C₁ à C₄, un halogène ou un perfluoroalkyle en C₁ à C₄, ou encore représente un naphtyle,
W représente une liaison ou un atome d'oxygène,
A³ représente une liaison ou un alkylène en C₁ à C₂₀, éventuellement mono- ou bi-substitué par un phényle, un naphtyle, un alkyle en C₁ à C₄, ou un cycloalkyle en C₅ à C₇,
Y représente une liaison ou un atome d'oxygène, et
n représente un nombre entier de 0 à 3, et
R² représente un atome d'hydrogène, un alkyle en C₁ à C₄, un alcoxy en C₁ à C₄, ou un halogène, ou
A¹ et R², conjointement avec les atomes de carbone auxquels ils sont liés, représentent un groupe cycloalkyle en C₅ à C₇, dont les atomes de carbone hybridés sp3 sont éventuellement remplacés une ou deux fois par un atome d'oxygène,
A^{2'} représente un groupe n-propylène éventuellement monosubstitué par un alkyle en C₁ à C₁₂, un alkyl en C₁ à C₁₂-phényle ou un alkyl en C₁ à C₁₂₋oxyphényle,
ainsi que les solvates et/ou hydrates de composés de formule Ig destinés à être utilisés en tant que médicaments.

8. Composés selon la revendication 7, choisis dans le groupe constitué de la 6,6,6-trifluoro-1-(4-méthoxyphényl)hexan-1,5-dione la 6,6,6-trifluoro-1-(4-(4-phénoxybutoxy)phényl)hexan-1,5-dione ; la 6,6,6-trifluoro-1-(4-(3-phénylpropoxy)phényl)hexan-1,5-dione ; la 1-(4-bromophényl)-6,6,6-trifluorohexan-1,5-dione ; la 6,6,6-trifluoro-1-(4-(1-naphtyl)phényl)hexan-1,5-dione ; la 6,6,6-,trifluoro-1-(5,6,7,8-tétrahydronaphtalén-2-yl)hexan-1,5-dione ; la 6,6,6-trifluoro-1-(4-(4-méthoxy-1-naphtyl)phényl)hexan-1,5-dione ; la 6,6,6-trifluoro-1-(4-(2-naphtyl)phényl)hexan-1,5-dione ; la 6,6,6-trifluoro-1-(4-(hexadécyloxy)phényl)hexan-1,5-dione, et la 6,6,6-trifluoro-1-(4-(tétradécyloxy)phényl)hexan-1,5-dione.

9. Composés de formule générale Id, 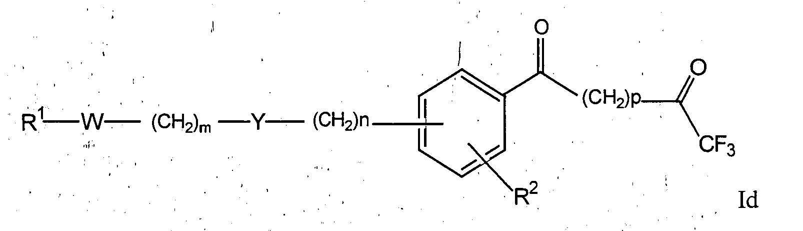 destinés à être utilisés en tant que médicaments selon la revendication 7,
dans laquelle
R¹ représente un atome d'hydrogène, un alkyle en C₁ à C₄, un cycloalkyle en C₃ à C₇, un alkyl en C₀ à C₄-phényle, éventuellement substitué dans le cycle phényle par un alkylènedioxy en C₁ à C₄, ou mono- ou bi-substitué par un alkyle en C₁ à C₄, un alcoxy en C₁ à C₄, un halogène ou un perfluoroalkyle en C₁ à C₄,
ou encore représente un naphtyle,
R² représente un atome d'hydrogène, un alkyle en C₁ à C₄, un alcoxy en C₁ à C₄, ou un halogène,
W représente une liaison ou un atome d'oxygène,
Y représente une liaison ou un atome d'oxygène, et
m représente un nombre de 0 à 10,
n représente un nombre entier compris entre 0 à 3, et
p'vaut 3.

10. Composés destinés à être utilisés en tant que médicaments, choisis dans le groupe constitué de la 1,1,1-trifluoro-7-phényl-heptan-2-one et de la 5-(4-méthoxyphényl)-1,1,1-trifluoro-pentan-2-one.

11. Procédé de préparation de composés de formule générale I", 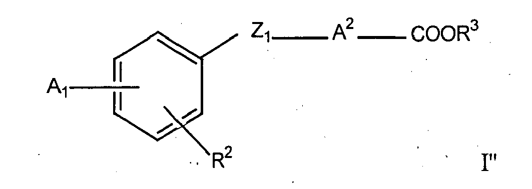 dans laquelle
A¹ représente un groupe de formule R¹-W-A³-Y-(CH₂)ₙ, où
R¹ représente un atome d'hydrogène, un alkyle en C₁ à C₄, un cycloalkyle en C₃ à C₇, un alkyl en C₀ à C₄-phényle; éventuellement substitué dans le cycle phényle par un alkylènedioxy en C₁ à C₄, ou mono- ou bi-substitué par un alkyle en C₁ à C₄, un alcoxy en C₁ à C₄, un halogène ou un perfluoroalkyle en C₁ à C₄,
ou encore représente un naphtyle,
W représente une liaison ou un atome d'oxygène,
A³ représente une liaison ou un alkylène en C₁ à C₂₀, éventuellement mono- ou bi-substitué par un phényle, un naphtyle, un alkyle en C₁ à C₄, ou un cycloalkyle en C₅ à C₇,
Y représente une liaison ou un atome d'oxygène, et
n représente un nombre entier de 0 à 3, et
R² représente un atome d'hydrogène, un alkyle en C₁ à C₄, un alcoxy en C₁ à C₄, ou un halogène, ou
A¹ et R², conjointement avec les atomes de carbone auxquels ils sont liés, représentent un groupe cycloalkyle en C₅ à C₇, dont les atomes de carbone hybridés sp3 sont éventuellement remplacés une ou deux fois par un atome d'oxygène,
Z' représente un groupe carbonyle et
A^{2"} représente un groupe alkylène en C₁ à C₃, éventuellement monosubstitué par un alkyle en C₁ à C₁₂, un alkyl en C₁ à C₁₂-phényle ou un alkyl en C₁ à C₁₂-oxyphényle,
**caractérisé en ce que** l'on fait réagir un composé de formule générale XIb", 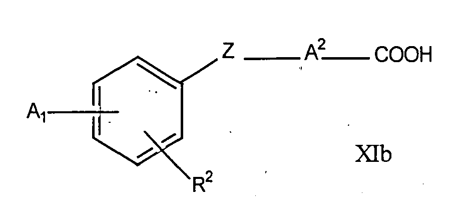 dans laquelle A¹, A^{2"}, R² et Z' ont les significations ci-dessus, avec un l'anhydride d'acide trifluoroacétique ou un anhydride d'acide acétique et **en ce que** l'on fait réagir les enlactones cycliques obtenues en tant que produits intermédiaires avec du trifluorométhyltriméthylsilane.
